# FASCICULE DE BREVET EUROPEEN

(11) **EP 1 853 712 B1**
(45) Date de publication et mention de la délivrance du brevet: **22.11.2017**
(21) Numéro de dépôt: 06709310.4
(22) Date de dépôt: 14.02.2006
(51) Int. Cl.: C12N 15/869, C12N 15/11

(54) **VECTEURS D'EXPRESSION STABLE ET DE LONGUE DUREE DE SIRNA ET LEURS APPLICATIONS**
STABILE SIRNA EXPRESSIONSVEKTOREN UND DEREN ANWENDUNGEN
STABLE AND LONG-LASTING SIRNA EXPRESSION VECTORS AND THE APPLICATIONS THEREOF

(30) Priorité: 14.02.2005 FR 0501483
(43) Date de publication de la demande: 14.11.2007
(73) Titulaire: Commissariat à l'Énergie Atomique et aux Énergies Alternatives, 75015 Paris (FR)
(72) Inventeur: BIARD, Denis, F-94320 Thiais (FR); ANGULO-MORA, Jaime F., F-91470 Limours (FR)
(74) Mandataire: Gevers & Orès
(86) Numéro de dépôt international: PCT/FR2006/000330
(87) Numéro de publication internationale: WO 2006/085016

(56) Documents cités:
- EP-A- 1 484 393
- BIARD DENIS S F ET AL: "Development of new EBV-based vectors for stable expression of small interfering RNA to mimick human syndromes: application to NER gene silencing." MOLECULAR CANCER RESEARCH : MCR. SEP 2005, vol. 3, no. 9, septembre 2005 (2005-09), pages 519-529, XP002349462 ISSN: 1541-7786
- MIYAGISHI, MAKOTO ET AL: "Expression of siRNA from a pol III promoter in mammalian cells" 2003, PERSPECTIVES IN GENE EXPRESSION , 361-375,A35, 1 PLATE. EDITOR(S): APPASANI, KRISHNARAO. PUBLISHER: EATON PUBLISHING CO., WESTBOROUGH, MASS. CODEN: 69FDMF; ISBN: 1-881299-16-3 , XP001246949 le document en entier
- BRUMMELKAMP T R ET AL: "A system for stable expression of short interfering RNAs in mammalian cells" SCIENCE, AMERICAN ASSOCIATION FOR THE ADVANCEMENT OF SCIENCE,, US, vol. 296, no. 5567, 2002, pages 550-553, XP002225638 ISSN: 0036-8075
- DATABASE GOOGLE [Online] 22 juin 2006 (2006-06-22), XP002386946 extrait de HTTP://WWW.INVITROGEN.COM/CONTENT/SFS/VECT ORS/PCDNA3.PDF
- BIARD D S F ET AL: "REGULATION OF THE ESCHERICHIA-COLI LAC OPERON EXPRESSED IN HUMAN CELLS" BIOCHIMICA ET BIOPHYSICA ACTA, vol. 1130, no. 1, 1992, pages 68-74, XP009055483 ISSN: 0006-3002
- BIARD D S F ET AL: "ESTABLISHMENT OF A HUMAN CELL LINE FOR THE DETECTION OF DEMETHYLATING AGENTS" EXPERIMENTAL CELL RESEARCH, vol. 200, no. 2, 1992, pages 263-271, XP009055400 ISSN: 0014-4827
- CHITTENDEN T ET AL: "FUNCTIONAL LIMITS OF ORIP, THE EPSTEIN-BARR VIRUS PLASMID ORIGIN OF REPLICATION" JOURNAL OF VIROLOGY, NEW YORK, US, US, vol. 63, no. 7, juillet 1989 (1989-07), pages 3016-3025, XP002914212 ISSN: 0022-538X
- D'HEROUEL A F ET AL: "FR-like EBNA1 binding repeats in the human genome", VIROLOGY, ELSEVIER, AMSTERDAM, NL, vol. 405, no. 2, 30 September 2010 (2010-09-30), pages 524-529, XP027459053, ISSN: 0042-6822, DOI: 10.1016/J.VIROL.2010.06.040 [retrieved on 2010-09-30]
- VAN CRAENENBROECK K ET AL: "Orientation-dependent gene expression with Epstein-Barr virus-derived vectors", FEBS LETTERS, ELSEVIER, AMSTERDAM, NL, vol. 555, no. 3, 18 December 2003 (2003-12-18), pages 489-494, XP004481037, ISSN: 0014-5793, DOI: 10.1016/S0014-5793(03)01311-5

### SEQUENCE LISTING

<110> commissariat à l'Energie Atomique
   BIARD, Denis
   ANGULO-MORA, Jaime F
<120> VECTEURS D'EXPRESSION STABLE ET DE LONGUE DURÉE DE siRNA ET LEURS APPLICATIONS
<130> BLOcp263/129
<150> FR 05 01483
   <151> 2005-02-14
<160> 19
<170> PatentIn version 3.3
<210> 1
   <211> 222
   <212> DNA
   <213> Artificial
<220>
   <223> promoteur H1
<400> 1
   ggaattcgaa cgctgacgtc atcaacccgc tccaaggaat cgcgggccca gtgtcactag     60
   gcgggaacac ccagcgcgcg tgcgccctgg caggaagatg gctgtgaggg acaggggagt    120
   ggcgccctgc aatatttgca tgtcgctatg tgttctggga aatcaccata aacgtgaaat    180
   gtctttggat ttgggaatct tataagttct gtatgagacc ac                       222
<210> 2
   <211> 24
   <212> DNA
   <213> Artificial
<220>
   <223> amorce sens H1
<400> 2
   ccatggaatt cgaacgctga cgtc 24
<210> 3
   <211> 44
   <212> DNA
   <213> Artificial
<220>
   <223> amorce anti-sens H1
<400> 3
   gcaagcttag atctgtggtc tcatacagaa cttataagat tccc 44
<210> 4
   <211> 19
   <212> DNA
   <213> Artificial
<220>
   <223> siRNA Kin17
<400> 4
   tcctcagcag tttatggat 19
<210> 5
   <211> 19
   <212> DNA
   <213> Artificial
<220>
   <223> siRNA XPC
<400> 5
   ggatgaagcc ctcagcgat 19
<210> 6
   <211> 19
   <212> DNA
   <213> Artificial
<220>
   <223> siRNA XPA
<400> 6
   gtcaagaagc attagaaga 19
<210> 7
   <211> 19
   <212> DNA
   <213> Artificial
<220>
   <223> siRNA XRCC4
<400> 7
   gagatccagt ctatgatga 19
<210> 8
   <211> 19
   <212> DNA
   <213> Artificial
<220>
   <223> siRNA Ku70
<400> 8
   gagtgaagat gagttgaca 19
<210> 9
   <211> 19
   <212> DNA
   <213> Artificial
<220>
   <223> siRNA DNA-PK
<400> 9
   gttgaggttc ctatggaaa 19
<210> 10
   <211> 19
   <212> DNA
   <213> Artificial
<220>
   <223> siRNA Rad50
<400> 10
   gaacttatag ctgtgcaaa 19
<210> 11
   <211> 19
   <212> DNA
   <213> Artificial
<220>
   <223> siRNA Rad51
<400> 11
   gaagaaattg gaagaagct 19
<210> 12
   <211> 19
   <212> DNA
   <213> Artificial
<220>
   <223> siRNA rad52
<400> 12
   gacaaagact acctgagat 19
<210> 13
   <211> 19
   <212> DNA
   <213> Artificial
<220>
   <223> SiRNA HR23B
<400> 13
   gcattaggat ttcctgaag 19
<210> 14
   <211> 19
   <212> DNA
   <213> Artificial
<220>
   <223> siRNA HR23A
<400> 14
   agacgatgct gacggagat 19
<210> 15
   <211> 19
   <212> DNA
   <213> Artificial
<220>
   <223> siRNA p53
<400> 15
   caagcaatgg atgatttga 19
<210> 16
   <211> 24
   <212> DNA
   <213> Artificial
<220>
   <223> amorce sens pBD751
<400> 16
   ggtaccagat ctgatccgaa atta 24
<210> 17
   <211> 30
   <212> DNA
   <213> Artificial
<220>
   <223> amorce anti-sens pBD751
<400> 17
   gtcgacaagc ttttgatcag atcggtgcgg 30
<210> 18
   <211> 65
   <212> DNA
   <213> Artificial
<220>
   <223> oligo 1
<400> 18
   agatcccgtc aagtactctg ggaccgttca agagacggtc ccagagtact tgactttttt 60
   ggaaa 65
<210> 19
   <211> 65
   <212> DNA
   <213> Artificial
<220>
   <223> oligo 2
<400> 19
   ggcagttcat gagaccctgg caagttctct gccagggtct catgaactga aaaaaccttt 60
   tcgaa 65

## Description

La présente invention est relative au domaine de l'interférence ARN et plus particulièrement à la mise au point de vecteurs d'expression stable et de longue durée d'ARN interférent (siRNA) et à leurs applications.

L'invention concerne donc également des cellules humaines ou non-humaines et des animaux transgéniques non-humains qui comprennent ledit vecteur ainsi que leurs applications pour l'évaluation de l'activité des siRNA et leur validation en tant que médicament ainsi qu'en thérapeutique humaine.

Il a été montré que des petits fragments d'ARN double-brin de 21 à 25 nucléotides, complémentaires d'un ARNm, sont capables, lorsqu'ils sont introduits dans des cellules eucaryotes, d'inhiber fortement l'expression du gène correspondant en détruisant cet ARNm (pour une revue, voir Biofutur (33), Voorhoeve et al. (34)). Ce phénomène d'inhibition ou d'extinction spécifique de l'expression des gènes, dénommé interférence ARN (RNAi), ouvre des perspectives intéressantes dans de nombreux domaines ; en particulier, dans le domaine de la génomique fonctionnelle et de la recherche pharmaceutique, les petits ARN interférents (*small interfering RNA* ou *silencing inducing RNA*), dénommés siRNA, sont utiles respectivement pour identifier la fonction de nouveaux gènes, et pour sélectionner les gènes cibles et les médicaments candidats. En outre, les siRNA peuvent également être utilisés directement comme médicaments.

Ainsi des siRNA spécifiques des transcrits codant pour des protéines virales ou cellulaires et capables d'inhiber la production des protéines correspondantes ont été décrits.

Plus précisément, le processus d'interférence ARN comprend essentiellement plusieurs étapes et semble initié par le clivage processif de longs segments d'ARN double-brin en fragments double-brin de 21 à 25 nucléotides (siRNA). Ces duplex de siRNA, produits par l'enzyme DICER (RNaseIII de la famille des dsRNA endonucléases spécifiques), sont incorporés dans un complexe protéique (RISC ou *RNA Induced Silencing Complex*) qui reconnaît et clive un ARN cible complémentaire d'un brin du siRNA et qui entraîne donc la dégradation d'un ARNm spécifique.

Les siRNA agissent à des concentrations faibles, et peuvent être exprimés par voie intracellulaire notamment à partir des promoteurs de l'ARN polymérase III (Pol III).

Il existe à l'heure actuelle trois approches différentes pour éteindre ou inhiber l'expression d'un gène dans des cellules de mammifère et notamment des cellules humaines, en utilisant la technique d'interférence ARN : (1) transfection de duplex siRNA, (2) transfection de vecteurs d'expression non viraux et (3) infection par des virus recombinants.

### (1) Duplex siRNA

De nombreux documents décrivent l'administration directe d'un duplex d'ARN double-brin (siRNA), notamment par transfection {(Soutschek J. et al. (27) ; Elbashir SM. et al. (3) ; Bantounas I. et al. (28)}. Bien que cette approche soit extrêmement efficace, elle présente les inconvénients suivants : (i) les duplex siRNA sont instables et généralement coûteux ; (ii) la transfection est transitoire et ne permet donc pas d'études à moyen et long terme ; (iii) le coût rend cette approche impossible si l'on veut travailler sur un très grand nombre de cellules (dans le cadre d'une étude de purification de complexes multi-protéiques, par exemple).

### (2) et (3) Vecteurs d'expression non viraux intégratifs et vecteurs viraux

La durée de vie courte des siRNA *in vivo* a conduit différentes équipes de chercheurs travaillant sur ce sujet à développer des systèmes à base de vecteurs qui devaient permettre une expression plus stable des siRNA dans les cellules de mammifère.

Dans ce but, certains Auteurs ont développé des vecteurs d'expression (plasmides) intégratifs (Barquinero J. et al. (30)) ou des vecteurs viraux (essentiellement rétrovirus, lentivirus ou adénovirus). Ces vecteurs ont l'avantage d'être adaptables. Ainsi, les cellules seront soit transfectées (plasmides) soit infectées (vecteurs viraux).

Ces vecteurs (plasmidiques ou viraux) utilisent la même stratégie d'expression de la séquence siRNA : à l'intérieur du vecteur, une séquence d'ADN va coder un ARN qui aura une structure en épingle à cheveux (shRNA *pour small hairpin RNA*). Il a aussi été envisagé des structures en tandem (*a priori* moins efficaces). Dans le cas de l'expression d'une structure shRNA, la stratégie mise en oeuvre implique, par exemple, le clonage d'une séquence sens correspondant au futur siRNA, suivi d'un bras espaceur ou boucle (*loop*) et de la séquence anti-sens dudit siRNA, suivie d'une série de résidus T (polyU dans le siRNA final). Lors de la synthèse *in situ* de cette séquence shRNA, la présence du bras espaceur entraînera la formation d'une structure en épingle à cheveux (shRNA) qui permettra l'appariement des séquences sens et anti-sens du futur siRNA. Cette structure en épingle à cheveux sera reconnue par une RNase III endogène (DICER) qui la coupera pour générer un siRNA fonctionnel.

Afin de permettre la transcription efficace de ces structures d'ARN de très petite taille, il a été préconisé d'utiliser des promoteurs spécifiques de l'ARN polymérase III (promoteurs Pol III), tels que les promoteurs U6, H1 ou ARNt. Il est à noter que l'ARN Pol III est spécialisée dans la transcription des petits ARN non codant comme les tRNA, lesquels présentent des sites de terminaison de la transcription parfaitement définis par des séquences de 4 à 6 thymidines (Sui G. et al. (35)). Cependant cela n'exclut pas l'utilisation de promoteurs spécifiques de l'ARN Pol II, comme le promoteur/amplificateur humain CMV IE (*cytomegalovirus immediate-early gene enhancer*/*promoter*) ou des promoteurs CMV modifiés comme ceux décrits dans la littérature (Dubois-Dauphin, 2004 (36) ; Hu et al. 2004 (37) ; Liu et al. 2004 (38), Sato et al. 2003 (39)).

Vecteur d'expression : Ainsi, Brummelkamp et al. (5) ont décrit un plasmide intégratif (pSUPER), qui comporte un promoteur Pol III HI capable de diriger la transcription d'ARN en épingle à cheveux (shRNA) pour conduire à la synthèse *in situ* d'un siRNA fonctionnel. Toutefois, ce plasmide intégratif présente les inconvénients suivants :
- Il ne permet d'obtenir qu'une inhibition ou extinction transitoire.
- Il est présent dans les cellules transfectées en très nombreuses copies par cellule ; ce qui peut saturer la machinerie RNAi (par exemple le complexe RISC) et provoquer des effets indésirables (6).
- Il s'intègre n'importe où dans le génome et peut donc induire, en sus des effets indésirables, des effets parasites.

Ce plasmide a ensuite été modifié pour y insérer un marqueur de sélection procaryote (généticine, par exemple). Cependant, la plupart des auteurs n'utilisent ces vecteurs que dans des essais de transfection transitoire, et seulement quelques rares clones ont été décrits après quelques jours de culture (7-9).

Vecteurs viraux : ces vecteurs permettent l'infection de cellules cibles difficiles à transfecter (Dykxhoorn D. et al. (29) ; Barquinero J. et al. (30)). De tels vecteurs viraux permettent un ciblage efficace et une expression *a priori* à moyen terme dans les cellules cibles. Leur intérêt dans des approches *in vivo* chez la souris a également été souligné. Cependant, le grand désavantage de ces virus recombinants est qu'ils posent de réels problèmes de sécurité car la plupart des laboratoires ne sont pas équipés pour travailler dans les règles de l'art avec ces virus. Leur utilisation pour évaluer des siRNA n'est donc pas toujours intéressante.

Ainsi, si l'on exclut les vecteurs viraux, pour des raisons de sécurité, les essais d'interférence ARN dans les cellules humaines en culture sont basés principalement sur des transfections transitoires d'ARN double-brin ou de plasmides intégratifs. Dans ce contexte, il est difficile d'isoler des clones stables. En conséquence, très peu de clones ont pu effectivement être maintenus en culture et ce uniquement pendant des périodes de temps très limitées (quelques jours) (7-9), alors qu'il existe un réel besoin d'une expression stable, pour obtenir une dérégulation soutenue de la cible en vue d'une évaluation à long terme des siRNA.

La présente Demande s'est en conséquence donnée pour but de pourvoir à des vecteurs qui augmentent de manière significative l'efficacité de l'ARNi, lesdits vecteurs permettant d'obtenir une inhibition ou extinction efficace, spécifique et à très long terme d'un gène cible dans les cellules humaines.

En effet, il est important de disposer d'un système de validation performant des effets en aval médiés par des siRNAs, dans la mesure où les résultats obtenus en mettant en oeuvre des siRNA ne sont pas toujours ceux attendus (19), des siRNA pouvant notamment induire des effets non spécifiques au niveau protéique.

Pour éviter une telle situation, les Inventeurs ont mis au point une nouvelle construction qui répond mieux aux besoins de la pratique que les constructions de l'Art antérieur notamment :
- en ce qu'elle permet une expression stable des siRNA à très long terme (plusieurs mois) dans des cellules de mammifère en culture ;
- en ce qu'elle permet d'éviter les effets indésirables liés à la saturation de la machinerie cellulaire ;
- en ce qu'elle permet de disposer d'un outil d'étude à long terme (plusieurs mois) des conséquences phénotypiques de la perte (partielle ou totale) de l'expression d'un gène ; cela permet de tester des sondes siRNA et d'analyser les conséquences biologiques de ces sondes sur des cellules maintenues en culture très longtemps (plusieurs mois). Cela permet de mettre en oeuvre un procédé de validation de sondes siRNA avant leur introduction dans des vecteurs viraux construits à des fins thérapeutiques (rétrovirus, lentivirus ou adénovirus) ;
- en ce qu'elle permet de mimer des pathologies humaines sur des cellules maintenues longtemps en culture (modèle particulièrement plus souple que des cellules obtenues par knock-out) ; et
- en ce qu'elle permet d'obtenir, pour la première fois, des cellules humaines avec des génomes quasi-identiques, c'est-à-dire ne différant que par une petite séquence de shRNA.

L'objet de la présente invention est défini par les revendications 1 à 22.

L'invention concerne un vecteur d'expression d'un siRNA, capable d'inhiber ou d'éteindre l'expression d'un gène cible dans une cellule de mammifère, lequel vecteur est caractérisé en ce qu'il comprend :
(1) une cassette bactérienne comprenant une origine de réplication bactérienne et un marqueur de sélection bactérienne M1,
(2) une cassette de sélection dans les cellules eucaryotes comprenant un marqueur de sélection M2 de cellules eucaryotes et notamment de cellules de mammifère, sous le contrôle d'un promoteur approprié,
(3) une cassette EBV comprenant : (i) au moins un fragment de l'antigène nucléaire 1 du virus d'Epstein-Bar (EBNA-1) dont la séquence dérive du vecteur p205 dont 700 des 717 pb de la répétition Gly-Gly-Ala du virus B95-8 ont été délétés, (ii) au moins un fragment de la famille de répétition (FR) comprenant entre 6 et 20 copies du motif de 30 pb, chaque copie contenant un site d'association à la protéine virale EBNA-1, et des éléments de terminaison primaire de la réplication et (iii) la région de double symétrie (DYAD) et
(4) une cassette de transcription d'un siRNA comprenant au moins une région codant pour un siRNA correspondant au gène cible à inhiber ou à éteindre, sous le contrôle d'éléments régulateurs de transcription dans des cellules de mammifère, lesquels éléments régulateurs incluent au moins un promoteur capable de transcrire une séquence ADN codant pour un siRNA dans des cellules de mammifère et un terminateur de transcription ; ladite cassette de transcription est telle que la séquence codant pour ledit siRNA est immédiatement en aval du site d'initiation de la transcription ;
ledit vecteur étant caractérisé en ce que :
- ladite cassette de transcription d'un siRNA (4) comprend un promoteur H1,
- ladite région codant pour le siRNA est un shRNA, et
- ladite cassette de transcription d'un siRNA (4) et la cassette EBV (3) sont en orientation inverse.

La présente description a, en conséquence, pour objet un vecteur d'expression d'un siRNA, capable d'inhiber ou d'éteindre l'expression d'un gène cible (endogène ou exogène) dans une cellule de mammifère, lequel vecteur est caractérisé en ce qu'il comprend :
(1) une cassette bactérienne comprenant une origine de réplication bactérienne et un marqueur de sélection bactérienne M1,
(2) une cassette de sélection dans les cellules eucaryotes comprenant un marqueur de sélection M2 de cellules eucaryotes et notamment de cellules de mammifère, sous le contrôle d'un promoteur approprié,
(3) une cassette EBV comprenant au moins un fragment de l'antigène nucléaire 1 du virus d'Epstein-Bar (EBNA-1), au moins un fragment de la famille de répétition (FR) et au moins un fragment de la région de double symétrie (DYAD) et
(4) une cassette de transcription d'un siRNA comprenant au moins une région codant pour un siRNA correspondant au gène cible à inhiber ou à éteindre, sous le contrôle d'éléments régulateurs de transcription dans des cellules de mammifère, lesquels éléments régulateurs incluent au moins un promoteur capable de transcrire un siRNA dans des cellules de mammifère et un terminateur de transcription.

Selon un mode de réalisation avantageux dudit vecteur, la cassette bactérienne (1) comprend un marqueur de sélection bactérienne M1 sélectionné dans le groupe constitué par les marqueurs de résistance à un antibiotique (ampicilline, kanamycine, rifampicine, tétracycline) et les marqueurs d'auxotrophie (TRP1, URA3).

Dans un mode de réalisation, l'invention concerne un vecteur tel que décrit précédemment, caractérisé en ce que la cassette bactérienne (1) comprend un marqueur de sélection bactérienne M1 sélectionné dans le groupe constitué par les marqueurs de résistance à un antibiotique et les marqueurs d'auxotrophie.

Selon un autre mode de réalisation avantageux dudit vecteur, la cassette de sélection dans les cellules eucaryotes (2) comprend un marqueur de sélection eucaryote M2 sélectionné dans le groupe constitué par les marqueurs de résistance à un antibiotique (hygromycine, généticine, néomycine ou G418, puromycine, et zéocine, blasticidine).

Dans un mode de réalisation, l'invention concerne un vecteur tel que décrit précédemment, caractérisé en ce que la cassette de sélection dans les cellules eucaryotes (2) comprend un marqueur de sélection eucaryote M2 sélectionné dans le groupe constitué par :
- l'hygromycine et
- la puromycine.

Selon une disposition avantageuse de ce mode de réalisation, ledit marqueur de sélection M2 est sous le contrôle d'un promoteur convenable sélectionné dans le groupe constitué par le promoteur de la thymidine kinase du virus HSV et le promoteur du virus SV40.

Dans un mode de réalisation, l'invention concerne un vecteur tel que décrit précédemment, caractérisé en ce que ledit marqueur de sélection M2 est sous le contrôle d'un promoteur convenable, sélectionné dans le groupe constitué par le promoteur de la thymidine kinase du virus HSV et le promoteur du virus SV40.

Les éléments de la cassette EBV (3) sont plus précisément les suivants :

### L'antigène nucléaire EBNA.1.

La protéine EBNA-1 comporte 641 acides aminés avec une séquence primaire inhabituelle caractérisée par la présence de nombreuses répétitions. L'une d'entre elles (acides aminés 90 à 328) contient exclusivement des répétitions Gly-Gly-Ala. L'intégralité de la séquence EBNA-1 n'est pas nécessaire ; ainsi les vecteurs EBV selon la description dérivent du vecteur p205 de Yates et al. (57) dont 700 des 717 pb de la répétition Gly-Gly-Ala du virus B95-8 ont été délétées. Ces vecteurs se répliquent plus efficacement que leurs homologues portant l'intégralité du gène EBNA-1.

**L'origine de réplication de la phase d'infection latente (OriP) :** L'oriP est une région de 1.800 pb qui contient 2 séquences *en cis* séparées de 960 pb (Yates et al. (40), Sugden et al. (41)) : la région 1 ou **famille de répétitions** (séquence **"FR")** et la région 2 ou **séquence de double symétrie** (séquence **Dyad).**

### La famille de répétitions ("FR").

La séquence FR est composée de 20 copies presque parfaites d'un motif de 30 pb, chaque copie contenant un site d'association à la protéine virale EBNA-1. La séquence consensus d'association à EBNA-1 est (Rawlins et al. 1985 (42)) :
_{5'}AGATTA **GGATAGCT.ATGCTACCC** AGATAT_{3'}

Dans des vecteurs d'expression, en présence exclusivement de la protéine EBNA-1, la région FR se comporte comme un **amplificateur puissant de la transcription** pouvant augmenter l'activité d'un promoteur faible comme HSV-TK d'un facteur 200 (Reisman et al. 1986 (43)). Par ailleurs, la région FR intervient dans l'efficacité de la réplication et dans la détermination de la spécificité cellulaire. Si elle est absolument nécessaire à la réplication, cette séquence seule en présence d'EBNA-1 ne permet pas la réplication des plasmides EBV (Lupton et al. 1985 (44) ; Reisman et al. 1985 (45)).

Les fonctions d'amplificateur de la transcription et de maintenance du génome sous forme épisomale sont un processus hautement coopératif entre les différentes régions du FR, qui implique une séquence minimale de 6 à 7 répétitions du motif de 30 pb (Wysokenski et al. 1989 (46)). Un nombre plus faible de motifs de répétition entraîne une perte du maintien épisomal des plasmides ainsi qu'un important processus de réarrangement structurel conduisant à la formation de vecteurs recombinants de haut poids moléculaire, formes multimères tête-queue du plasmide d'origine. Ces plasmides ont retrouvé un nombre de motifs de répétition suffisant à leur maintien extrachromosomique (Chittenden et al. 1989 (47)).

Le motif FR contient des éléments de **terminaison primaire de la réplication** qui agissent, après la fixation d'EBNA-1, comme une barrière pour la fourche de réplication (Krysan et al. 1991 (48)). D'autre part, la séquence FR confère aux plasmides la propriété de rétention dans le nucléoplasme même en l'absence de leur réplication (Krysan et al. 1989 (49)).

### La région de double symétrie (Dyad).

Cette région de 140 pb contient 2 sites d'association à EBNA-1, localisés symétriquement à l'intérieur d'une région de 65 pb de double symétrie qui forment une structure en tige (31 pb)/boucle (3 pb). Deux autres sites encadrent cette région.

L'association de la protéine EBNA-1 avec la séquence Dyad facilite **l'initiation de la réplication** près de cet élément (Rawlins et al 1985, précité ; Reisman et al., 1985, précité ; Gahn et al. (50)). Cette origine de réplication est dépourvue de structure nucléosomique (Sexton et al. (51)), notamment à son extrémité 3'. La délétion de l'extrémité 3' de la séquence Dyad ou de sa totalité abolit complètement la maintenance à l'état d'épisomes des génomes EBV (Chittenden et al., 1989, précité).

La proximité des éléments de terminaison (FR) et d'initiation de la réplication (Dyad) dans l'oriP résulte dans une **réplication** principalement **unidirectionnelle** de l'ADN dans les vecteurs EBV. La fourche progressant vers la séquence FR est stoppée à ce niveau tandis que celle se dirigeant dans la direction opposée parcourt le plasmide pour rencontrer la fourche en attente dans le FR (50). Par ailleurs, il a été proposé que la direction de la transcription et le mouvement de la fourche de réplication puissent être coordonnés.

Selon un autre mode de réalisation avantageux dudit vecteur, le promoteur capable de transcrire un siRNA dans des cellules de mammifère est un promoteur de l'ARN polymérase III (type 1, 2 ou 3) ou un promoteur reconnu par l'ARN polymérase II comme le promoteur/amplificateur humain CMV IE (*cytomegalovirus immediate-early gene enhancer*/*promoter*) ou des promoteurs CMV modifiés comme ceux décrits dans la littérature (Dubois-Dauphin et al. (36) ; Hu et al. (37) ; Liu et al. (38) ; Sato et al. (39)).

Il est lié de manière opérationnelle avec la région codant pour le siRNA. Les séquences codant pour le siRNA sont immédiatement en aval du site d'initiation de la transcription (CCG, par exemple) ou bien distantes au maximum de ce dernier d'au plus 20 paires de bases. D'autres séquences de régulation peuvent avantageusement être associées audit promoteur : séquences inductibles ou modulables (par la tétracycline, par exemple Tet^{ON/OFF}, par l'IPTG ou le lactose, par exemple l'opérateur lacO, par la température ou par des génotoxiques chimiques ou physiques) ou séquences tissu-spécifique en choisissant éventuellement des promoteurs des ARN polymérases de type II ; dans ce cas on pourra privilégier des séquences hybrides shRNA-miR, dans lesquelles une structure de type shRNA est introduite dans la structure d'un microARN (miRNA) permettant la synthèse des siRNA par l'intermédiaire de promoteurs dépendant de l'ARN polymérase de type II (Sylva et al., Second-generation shRNA libraries covering the mouse and human genomes. Nat Genet. 2005; 37, 1281-1288).

Ladite cassette de transcription comprend en outre :
- en aval de la séquence codant pour le siRNA, un terminateur de transcription, qui comprend, de préférence, une séquence de thymidines, de manière préférée 4-6 thymidines et de manière encore plus préférée 6 thymidines consécutives dans le brin sens de la construction et
- un système de sélection pour le clonage ; à cet effet, un promoteur procaryote (T7, EM7...), suivi du fragment alpha du gène *LacZ* (*LacZ'* ou *LacZ alpha*) est introduit avantageusement dans la construction et permet la sélection dans la bactérie. Après complémentation dans des bactéries hôtes choisies à cet effet, les bactéries portant le vecteur d'origine seront bleues, celles portant un vecteur recombinant où le LacZ' a été remplacé par la séquence shRNA seront blanches.

Ce système constitue un critère de sélection lors de l'insertion d'une séquence shRNA.

Selon une disposition avantageuse de ce mode de réalisation, ledit promoteur de l'ARN polymérase III est sélectionné dans le groupe constitué par les promoteurs H1 RNA, U6 snRNA, 7SK RNA, 5S, adénovirus VA1, Vault, ARN télomérase et ARNt (Val, Met ou Lys3).

Le promoteur est de préférence le promoteur H1 ; ce promoteur contrôle l'expression d'ARN H1, qui est le composant ARN de la RNAse P humaine.

La séquence retenue pour le promoteur H1 est la suivante : et correspond à la séquence 145-366 du gène H1 et de son promoteur, référencé sous le n° d'accession X16612, dans la base de données NCBI.

La présente description concerne ainsi différentes constructions capables de produire des siRNAs. Dans ces constructions, les cassettes (1) et (2) contenant respectivement le marqueur de sélection bactérienne M1 et le marqueur de sélection eucaryote M2 sont de préférence dans la même orientation.

On entend par construction, au sens de la présente description, aussi bien des polynucléotides que des vecteurs.

Les constructions selon la description sont des constructions réplicatives qui ne saturent pas la machinerie RNAi et qui induisent une extinction du gène cible dans plus de 95% des cellules, deux semaines après la transfection (cela dépend toutefois de la séquence siRNA qui a été choisie), alors que des cellules transfectées avec des plasmides intégratifs (portant un marqueur de sélection) induisent moins de 50 % d'extinction, dans les mêmes conditions.

En outre, et ce de manière surprenante, elles maintiennent une extinction du gène cible, dans différents types de cellules de mammifère (HeLa, RKO, MRC5-V2, par exemple) en culture pendant des périodes supérieures à 1 an (très bonne reproductibilité).

La région codant pour le siRNA correspond à une molécule d'ARN double-brin qui comprend une région identique au gène cible. Le siRNA est capable d'éteindre ou d'inhiber le gène cible, de manière spécifique. De nombreuses publications fournissent les informations nécessaires pour sélectionner un siRNA {Gilmore IR et al. (31) ; Dykxhoorn D. et al. précité (29) ; Chalk AM et al. (52)}.

La construction selon la description génère, de préférence, un siRNA à partir d'un ARN précurseur en épingle à cheveux (shRNA).

Les constructions selon la description peuvent être introduites dans les cellules cibles par différentes méthodes de transfection :
- soit elles sont associées à une substance lui permettant de franchir la membrane des cellules cibles : transporteur (nanotransporteur), préparation de liposomes, polymères cationiques ou calcium (méthode chimique)
- soit elles sont introduites dans les cellules par une méthode physique : électroporation ou microinjection
- soit une association des deux méthodes est mise en oeuvre.

Les gènes cibles peuvent être n'importe quel gène d'intérêt.

Le but de l'extinction du gène peut être aussi bien thérapeutique que pour étudier la fonction d'un gène particulier ou l'ensemble des implications liées à l'extinction dudit gène. L'extinction peut ainsi modifier un phénotype.

Dans la présente description quatorze gènes cibles ont déjà été testés dont cinq ont plus particulièrement été étudiés (XPC, XPA, kin17, DNA PKcs et XRCC4). L'ensemble de ces études montrent l'intérêt des constructions selon la description.

Deux de ces gènes ont été choisis de telle sorte que leur extinction mime un syndrome humain et ce pour évaluer les conséquences à long terme de l'extinction de ces gènes dans des cellules syngéniques.

Les protéines XPA (numéro d'accès Genbank NM-00380) et XPC (numéro d'accès Genbank NM-004628) sont nécessaires à la reconnaissance et à l'excision de lésions d'ADN volumineuses au cours de la réparation par excision-resynthèse de nucléotides (NER). Ces protéines sont associées aux pathologies suivantes : sensibilité héréditaire au soleil, *xeroderma pigmentosum.*

La protéine XPC est essentielle pour l'étape initiale de reconnaissance de la lésion de l'ADN pendant la réparation globale du génome (*Global Genome Repair* ou GGR) (12). La protéine XPA participe à la formation d'un complexe de pré-incision (13).

Dans la mesure où il existe une interaction génétique entre les protéines XPA/XPC et la protéine kin17 (numéro d'accès Genbank NM-012311) (_{HSA}KIN17) (14), des vecteurs d'extinction du gène *KIN17* ont également été préparés. Le gène *KIN17* code pour une protéine nucléaire en doigt de zinc, impliquée dans la réplication de l'ADN et dans la réponse cellulaire aux lésions de l'ADN (15).

La protéine humaine XRCC4 (numéro d'accès Genbank NM_022550) est l'homologue à la protéine de rongeur « X-ray repair cross-complementing protein 4 ». La protéine DNA PKcs correspond à la sous-unité catalytique de la protéine kinase dépendant de l'ADN (DNA PK ; numéro d'accès Genbank NM_006904). XRCC4 agit en concert avec l'ADN ligase IV et avec la DNA PK dans les processus de réparation des cassures double chaînes de l'ADN par recombinaison NHEJ ou recombinaison V(D)J. Il est à noter que la protéine XRCC4 est essentielle à la survie cellulaire et qu'il n'existe pas de mutant naturel.

Ces deux protéines sont donc des éléments essentiels d'une voie de réparation de l'ADN indépendante de la voie associée aux protéines XPA et XPC.

La présente invention a également pour objet des cellules eucaryotes, de préférence des cellules de mammifère, modifiées par un vecteur tel que défini ci-dessus.

La présente invention a également pour objet un animal transgénique non-humain, caractérisé en ce qu'il comprend des cellules modifiées par un vecteur tel que défini ci-dessus.

La présente description a également pour objet l'utilisation d'un vecteur tel que défini ci-dessus ou d'une cellule telle que définie ci-dessus, pour inhiber ou éteindre l'expression d'un gène.

La présente invention a également pour objet l'utilisation *in vitro* d'un vecteur tel que défini ci-dessus ou d'une cellule telle que définie ci-dessus, pour inhiber ou éteindre l'expression d'un gène.

La présente invention a également pour objet une composition pharmaceutique, caractérisée en ce qu'elle comprend un vecteur tel que défini ci-dessus, et au moins un véhicule pharmaceutiquement acceptable.

La présente invention a aussi pour objet un procédé d'évaluation de l'activité d'un siRNA apte à inhiber ou éteindre l'expression d'un gène cible, lequel procédé est caractérisé en ce qu'il comprend les étapes suivantes :
(a₀) la transfection d'au moins une cellule de mammifère avec un vecteur selon l'invention,
(b₀) la mise en culture desdites cellules dans un milieu apte à sélectionner les cellules résistantes au marqueur de sélection eucaryote M2 et
(c₀) l'analyse du phénotype desdites cellules résistantes par comparaison avec le phénotype de cellules non transfectées.

La présente invention a également pour objet un procédé d'évaluation de l'activité d'un ensemble de siRNA conçus pour inhiber ou éteindre l'expression d'un gène cible, lequel procédé comprend :
(a₁) la préparation d'une banque de vecteurs selon l'invention dans lesquels différents siRNA, ayant le même gène cible, sont insérés,
(b₁) la transfection de cellules avec lesdits vecteurs de ladite banque ; chaque cellule ou chaque série de cellules étant transfectée avec un vecteur différent,
(c₁) la mise en culture desdites cellules dans un milieu apte à sélectionner les cellules résistantes au marqueur de sélection eucaryote M2 et
(d₁) l'analyse des différents phénotypes desdites cellules résistantes.

Selon un mode de mise en oeuvre avantageux desdits procédés d'évaluation de l'activité d'un siRNA, l'étape (c₀) ou l'étape (d₁) d'analyse du phénotype comprend la détection et/ou la quantification de l'expression du gène cible.

Selon un autre mode de mise en oeuvre avantageux desdits procédés d'évaluation de l'activité d'un siRNA, lesdits procédés de l'invention comprennent en outre, après l'étape (c₀) ou après l'étape (d₁), une étape (c'₀) ou une étape (d'₁) de mise en culture desdites cellules dans un milieu dépourvu de l'élément permettant la sélection des cellules résistantes au marqueur de sélection M2 et l'évaluation de la restauration du phénotype de départ par réversion.

La présente invention a pour objet l'utilisation d'une cellule selon l'invention, pour évaluer l'activité globale d'un siRNA.

En effet, les cellules selon la description qui peuvent être maintenues longtemps en culture permettent de tester les sondes siRNA et d'analyser les conséquences biologiques de ces sondes dans leur globalité ; cela permet en particulier de valider des sondes siRNA avant de décider de les utiliser effectivement comme médicament.

La présente invention a également pour objet un procédé de criblage de molécules aptes à traiter une maladie comprenant l'inhibition ou l'extinction d'un gène exprimant une protéine, lequel procédé comprend :
(a₂) la préparation d'un modèle d'étude de ladite maladie par la transfection d'au moins une cellule de mammifère avec un vecteur selon l'invention, comprenant un siRNA capable de mimer ladite maladie,
(b₂) la mise en culture desdites cellules dans un milieu apte à sélectionner les cellules résistantes au marqueur de sélection eucaryote M2,
(c₂) l'analyse du phénotype desdites cellules résistantes par comparaison avec le phénotype desdites cellules avant transfection,
(d₂) l'ajout dans le milieu de culture de la substance ou de la banque de substances à cribler,
(e₂) l'évaluation de la modification du phénotype desdites cellules et
(f₂) la sélection des molécules restaurant un phénotype normal et donc aptes à traiter ladite maladie.

Le phénotype analysé va dépendre du gène cible et des effets de son extinction.

La capacité d'évaluation du phénotype d'une cellule ou d'un organisme selon la description est importante dans le cadre des différents criblages, évaluations et validation de siRNA, tels que la capacité d'une molécule à moduler le phénotype d'une cellule ou d'un organisme.

De manière générale, on entend par phénotype, au sens de la présente description, une caractéristique observable d'un organisme ou d'une cellule. En d'autres termes, le phénotype est la manifestation de l'expression de gènes dans un organisme ou une cellule.

Plus précisément, un phénotype peut correspondre à n'importe quelle caractéristique d'une cellule ou d'un organisme qui résulte de l'interaction entre le profil génétique et l'environnement. Il peut s'agir de n'importe quelle manifestation d'un désordre ou d'une infection ; il peut également s'agir d'une caractéristique physiologique recherchée ; il peut inclure des caractéristiques biochimiques, moléculaires, cellulaires, tissulaires. L'évaluation peut être effectuée au niveau génétique, notamment en recherchant si l'expression d'un gène particulier, différent du gène ciblé par le siRNA étudié est modulé ou l'évaluation peut être effectuée par la détection de métabolites spécifiques de l'altération de l'expression génique obtenue, comme par exemple l'expression d'une protéine, de glucides ou de lipides ou d'autres molécules produites par la voie métabolique inactivée.

L'activité d'un récepteur, d'une protéine de transduction du signal, d'un canal membranaire ou d'une enzyme peut ainsi être analysée. Au niveau cellulaire, les fonctions suivantes peuvent être évaluées : migration, adhésion, dégranulation, phagocytose, apoptose, différenciation, chimiotaxie ; la transformation d'une cellule ou l'acquisition du caractère tumoral peuvent également être analysés.

Les différentes méthodes selon la description peuvent avantageusement être réalisées à haut débit dans des plaques à multi-puits.

Dans un autre aspect, l'invention concerne un produit intermédiaire pour la préparation d'un vecteur tel que décrit précédemment, caractérisé en ce qu'il comprend :
(1) une cassette bactérienne comprenant une origine de réplication bactérienne et un marqueur de sélection bactérienne M1, telle que définie précédemment,
(2) une cassette de sélection dans les cellules eucaryotes comprenant un marqueur de sélection M2 de cellules eucaryotes et notamment de cellules de mammifère, sous le contrôle d'un promoteur approprié, telle que définie précédemment,
(3) une cassette EBV comprenant (i) au moins un fragment de l'antigène nucléaire 1 du virus d'Epstein-Bar (EBNA-1) dont la séquence dérive du vecteur p205 dont 700 des 717 pb de la répétition Gly-Gly-Ala du virus B95-8 ont été délétés, (ii) au moins un fragment de la famille de répétition (FR) comprenant entre 6 et 20 copies du motif de 30 pb, chaque copie contenant un site d'association à la protéine virale EBNA-1, et des éléments de terminaison primaire de la réplication, et (iii) la région de double symétrie (DYAD), et
(4) le promoteur H1, suivi de deux sites de clonage, orienté en sens inverse par rapport à la direction de la cassette EBV.

La présente invention a, en outre, pour objet, un kit, caractérisé en ce qu'il comprend un vecteur tel que défini ci-dessus ou des cellules telles que définies ci-dessus et des moyens de détection et/ou de quantification de l'expression d'un gène cible (anticorps notamment, après western blot ou marquages immunocytochimiques).

La présente description a également pour objet un produit intermédiaire pour la préparation d'un vecteur tel que défini ci-dessus, caractérisé en ce qu'il comprend :
(1) une cassette bactérienne comprenant une origine de réplication bactérienne et un marqueur de sélection bactérienne M1,
(2) une cassette de sélection dans les cellules eucaryotes comprenant un marqueur de sélection M2 de cellules eucaryotes et notamment de cellules de mammifère, sous le contrôle d'un promoteur approprié, tel que défini ci-dessus,
(3) une cassette EBV comprenant au moins un fragment de l'antigène nucléaire 1 du virus d'Epstein-Bar (EBNA-1), au moins un fragment de la famille de répétition (FR) et au moins un fragment de la région de double symétrie (DYAD) et
(4) le promoteur H1 et deux sites de clonage sélectionnés dans l'une des deux orientations possibles.

Selon un mode de réalisation avantageux dudit produit intermédiaire, les deux sites de clonage sont Bgl II/Hind III.

Selon une disposition avantageuse de ce mode de réalisation dudit produit intermédiaire, le promoteur H1 et les sites de clonages sont en orientation directe et le marqueur de sélection M2 est l'hygromycine, ledit produit intermédiaire étant dénommé pBD665 ou pEBV-siD.

Selon une autre disposition avantageuse de ce mode de réalisation dudit produit intermédiaire, le promoteur H1 et les sites de clonages sont en orientation inverse (ou réverse) et le marqueur de sélection M2 est l'hygromycine, ledit produit intermédiaire étant dénommé pBD631 ou pEBV-siR.

Selon un autre mode de réalisation avantageux dudit produit intermédiaire, il comprend un système de sélection pour le clonage comprenant un promoteur procaryote, en particulier le promoteur procaryote T7, suivi du fragment alpha du gène LacZ (LacZ').

Selon une disposition avantageuse de ce mode de réalisation, le promoteur H1 et les sites de clonages sont en orientation inverse (ou réverse) et le marqueur de sélection M2 est l'hygromycine, ledit produit intermédiaire étant dénommé pBD751 ou pEBVsiR-LacZ' (ou pEBVsiR-LacZ'-Hygro).

Selon une autre disposition avantageuse de ce mode de réalisation, le promoteur H1 et les sites de clonages sont en orientation inverse (ou réverse) et le marqueur de sélection M2 est la puromycine, ledit produit intermédiaire étant dénommé pBD899 ou pEBVsiR-LacZ'-Puro.

Dans un mode de réalisation, l'invention concerne un produit intermédiaire tel que décrit précédemment, caractérisé en ce que le marqueur de sélection M2 est l'hygromycine, ledit produit intermédiaire étant dénommé pBD631 ou pEBV-siR.

Dans un mode de réalisation, l'invention concerne un produit intermédiaire tel que décrit précédemment, caractérisé en ce qu'il comprend un système de sélection pour le clonage comprenant un promoteur procaryote (T7) suivi du fragment alpha du gène LacZ (LacZ').

Dans un mode de réalisation, l'invention concerne un produit intermédiaire tel que décrit précédemment,, caractérisé en ce que le marqueur de sélection M2 est l'hygromycine, ledit produit intermédiaire étant dénommé pBD751 ou pEBVsiR-LacZ' (ou pEBVsiR-VacZ'-Hygro).

Dans un mode de réalisation, l'invention concerne un produit intermédiaire tel que décrit précédemment, caractérisé en ce que le marqueur de sélection M2 est la puromycine, ledit produit intermédiaire étant dénommé pBD899 ou pEBVsiR-LacZ'-Puro.

Ces deux derniers produits intermédiaires sont des vecteurs de clonage et permettent de cibler les bactéries qui portent un vecteur recombinant ; l'efficacité de clonage est proche de 100 %. En outre ces deux vecteurs de clonage pBD751 et pBD899 sont complémentaires et permettent de faire des doubles knock-down.

La présente invention a en outre pour objet un procédé de préparation d'un vecteur selon l'invention, caractérisé en ce qu'il comprend :
- la préparation d'un produit intermédiaire, tel que défini ci-dessus et
- l'insertion d'une séquence d'ADN codant un shRNA convenable au niveau des sites de clonage sélectionnés.

Outre les dispositions qui précèdent, la description comprend encore d'autres dispositions, qui ressortiront de la description qui va suivre, qui se réfère à des exemples de mise en oeuvre du procédé objet de la présente description ainsi qu'au Tableau illustrant les séquences de la Demande et aux dessins annexés, dans lesquels :

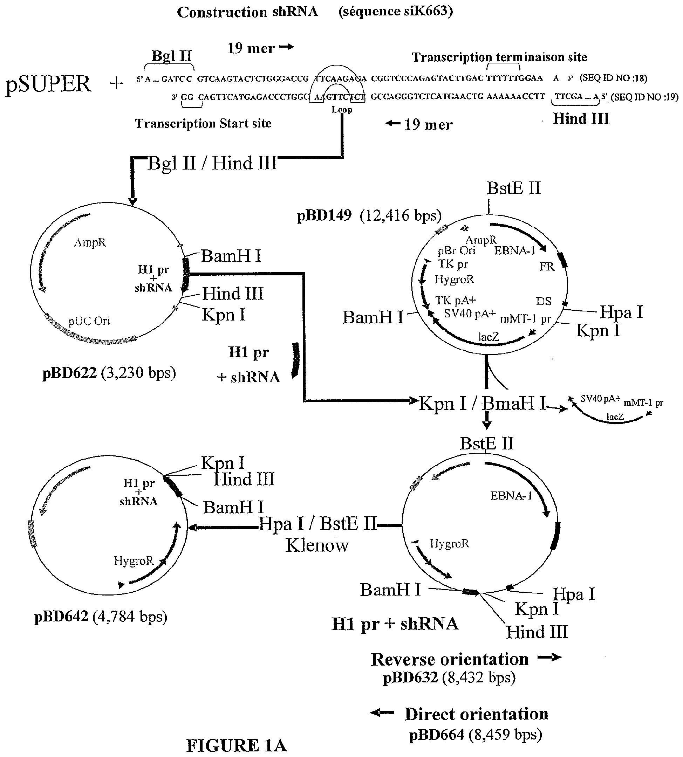

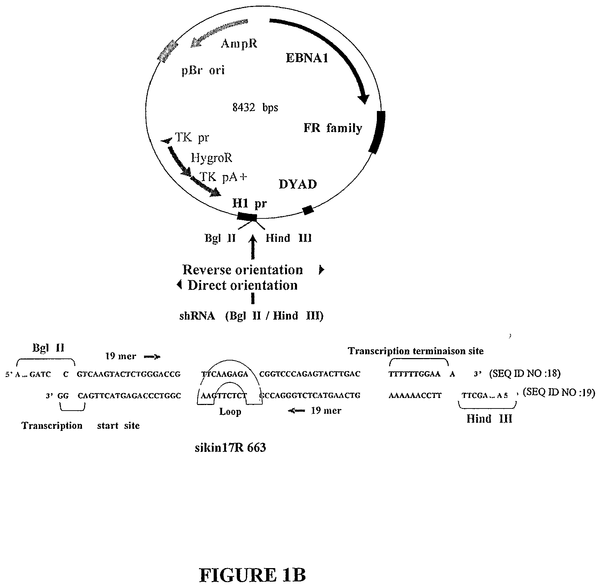

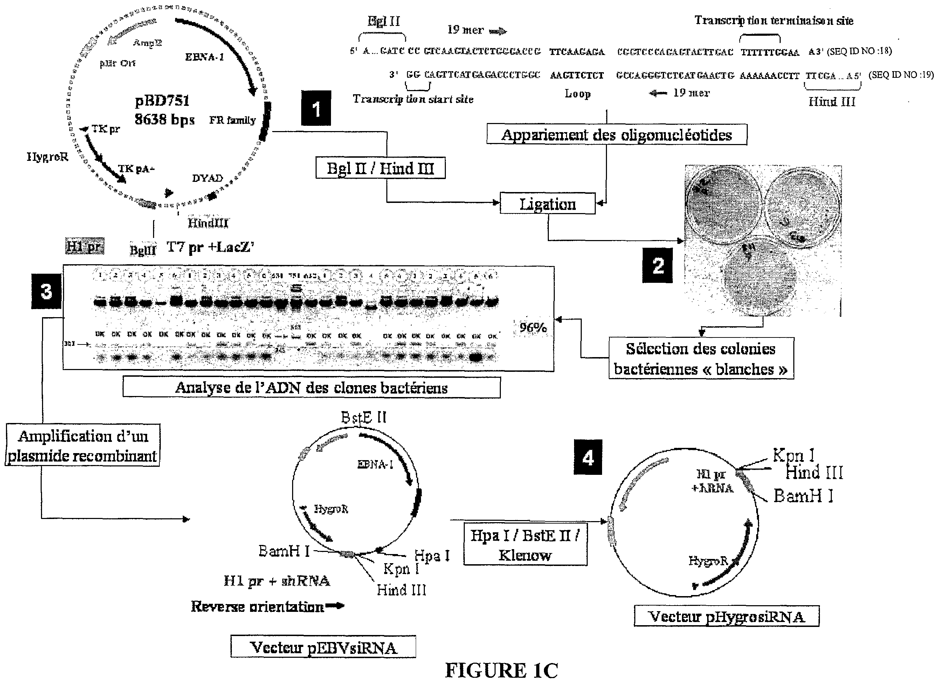

- Figure 1 : Carte génétique des vecteurs siRNA. Schéma de clonage. Plusieurs stratégies de clonage peuvent être envisagées ; le clonage indirect (Figure 1A) et le clonage direct (Figure 1C). A. Le domaine BamH I / Kpn I du vecteur pSUPER (5) contenant le site Bgl II, le promoteur H1, une séquence shRNA (ou non) et le site Hind III est inséré dans un vecteur EBV (pBD149) (Biard DS et al. (32)) donnant lieu au vecteur pBD631 (vecteur de clonage ne contenant pas de séquence shRNA et portant les sites Bgl II et Hind III intacts) ou aux vecteurs EBV-siRNA spécifiques d'un gène donné (portant une séquence shRNA spécifique et ayant perdu le site Bgl II lors du clonage).
   En comparaison, des plasmides intégratifs sont obtenus après délétion du segment EBV. Une stratégie similaire est utilisée pour obtenir une cassette siRNA en orientation inverse : FR : famille de séquences répétées ; DS : élément de symétrie Dyad ; HygroR : gène de résistance à l'hygromycine B ; TKpr : promoteur de la thymidine kinase du virus HSV ; TKpA+ : signal de polyadénylation HSV-TK ; pBrOri : origine de réplication pBR322 ; AMPr : gène de la β-lactamase bactérien. B. Cette figure illustre le vecteur pBD632 (= pEBV-siK663R), dans lequel la séquence siRNA *(KIN17)* est en orientation réverse :
   - Cassette EBV : EBNA-1+FR+DYAD
   - Cassette siRNA : H1 + shRNA
   - Cassette bactérienne : pBrOri + AmpR (=M1)
   - Cassette de résistance eucaryote : promoteur TK + Hygro (M2) + TKpA⁺.
      C. Le clonage direct : les oligonucléotides codant un shRNA sont introduits directement dans le vecteur de clonage pBD751 (pEBVsiR-LacZ') préalablement digéré par les enzymes de restriction Bgl II / Hind III (figure 1C1). L'insertion se fait en remplacement du fragment LacZ'. Après la ligation et la transformation de bactéries compétentes sur un milieu de culture « LB-agar + ampicilline + XGal / IPTG », on sélectionne les clones bactériens « blancs » résistant à l'ampicilline (Figure 1C2). 6 clones bactériens (voire moins) sont isolés et amplifiés, l'analyse de leur ADN plasmidique par différentes digestions enzymatiques (par exemple Hind III / BamH I) témoignera de l'efficacité de clonage qui est proche de 100 % (Figure 1C3). De même, l'obtention de vecteurs portant la résistance à la puromycine peut être obtenue à partir du vecteur de clonage pBD899 (pEBVsiR-LacZ'-Puro). Les plasmides intégratifs sont obtenus après délétion du segment EBV (digestion Hpa I / BstE II / remplissage Klenow et autoligation) (Figure 1C4).

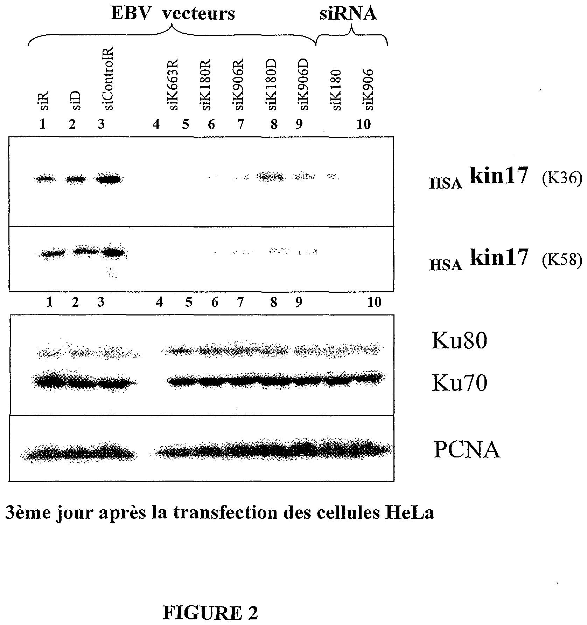

- Figure 2 : des cellules humaines HeLa sont mises en culture et transfectées 24 h après, alors qu'elles sont en croissance exponentielle, ce qui potentialise l'effet des siRNA ou des vecteurs associés. La transfection est faite avec 3 µl de LIPOFECTAMINE 2000 (Invitrogen) et soit 2 µg d'ADN (pour les vecteurs d'expression), soit 60 nM (concentration finale) de duplex siRNA. Trois jours après, les cellules sont trypsinisées, comptées et les protéines analysées.
   Anticorps :
   anti-_{HSA}kin17 : immunoglobines IgK36 et K58 purifiées à partir de fluides d'ascite et correspondant aux anticorps monoclonaux mAB K36 et mAB K58 (Biard et al. (58)).
   anti-Ku70 : Neomarkers, clone N3H10
   anti-Ku80 : Neomarkers, clone 111
   anti-cycline A : Sigma, clone CY-A1
   anti-α, tubuline : Sigma, clone B-5-1-2
   anti-PCNA : Novo Castra, clone PC10.

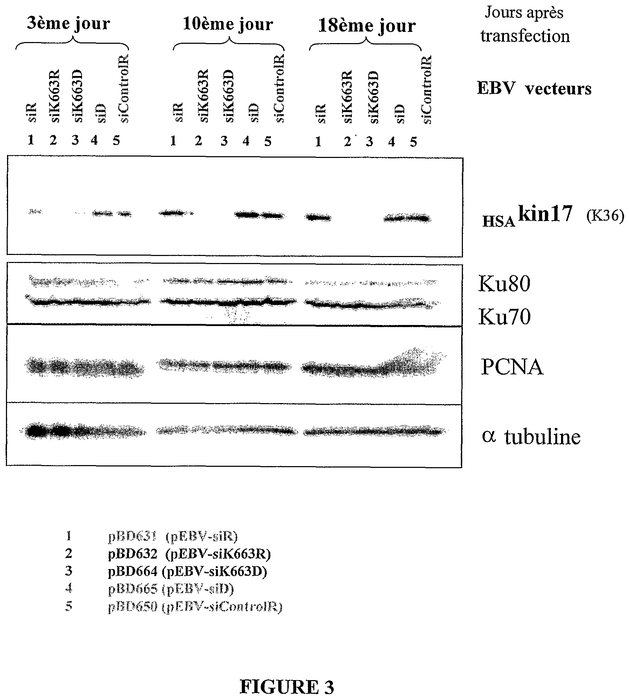

- Figure 3 : des cellules humaines HeLa sont transfectées avec 3 µl de LIPOFECTAMINE 2000 (Invitrogen) et soit 2 µg d'ADN, soit 60nM (concentration finale) de duplex siRNA. Les cellules HeLa transfectées sont mises en culture en présence de 250 µg/ml d'hygromycine B (Invitrogen) pendant 3, 10 et 18 jours.
   1 : pEBV-siR (= pBD631)
   2 : pEBV-siK 663R (= pBD632)
   3 : pEBV-siK 663D (= pBD664)
   4 : pEBV-siD (= pBD665)
   5 : pEBV-si control R (= pBD650)

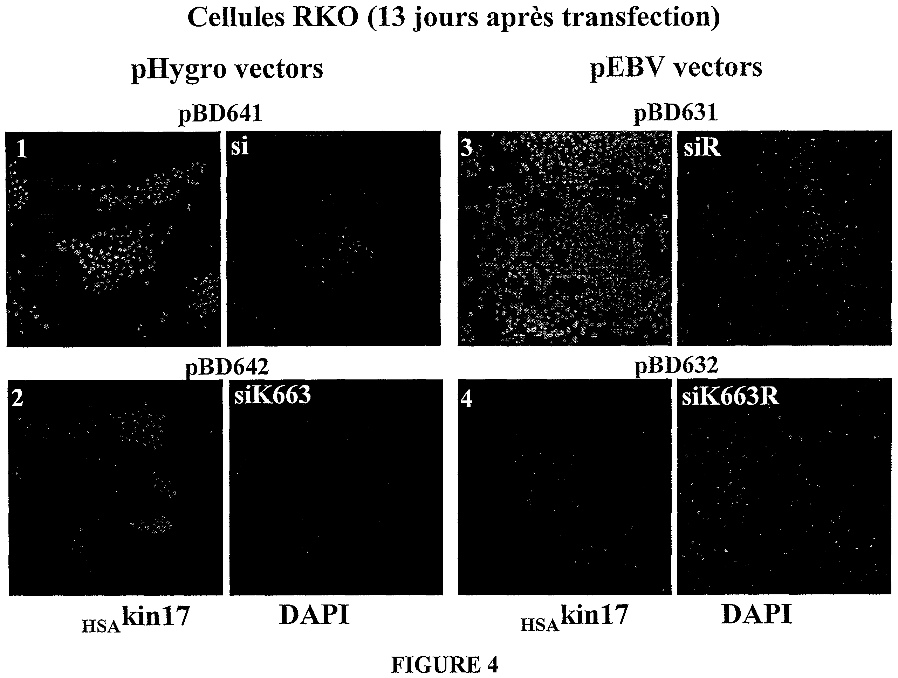

- Figure 4 : 48 h après la transfection, les cellules RKO sont ensemencées à raison de 5000 cellules/cm² en présence d'hygromycine B (500 µg/ml).
   La détection immunohistochimique de la protéine _{HSA}kin17 est effectuée 13 jours après la transfection (IgG K36 ; contremarquage DAPI ; agrandissement x50) :
   1 : pHygro-si (pBD641)
   2 : pHygro-siK 663 (pBD642)
   3 : pEBV-siR (= pBD631)
   4 : pEBV-siK 663R (= pBD632).

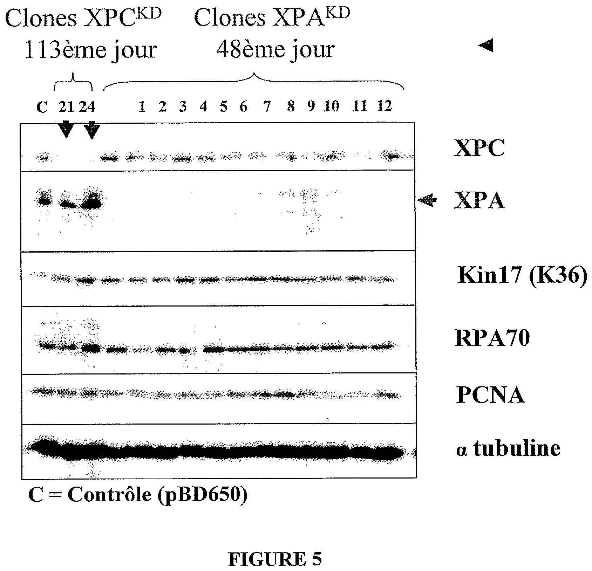

- la figure 5 illustre l'analyse en western blot de 12 clones stables XPA^{KD} (clones cellulaires devenus silencieux *-knock down-* pour XPA) et de deux clones stables XPC^{KD} (clones cellulaires devenus silencieux *-knock down-* pour XPC). Anticorps : anti-XPA (Yang ZG et al. (53)) et anti-XPC (Volker M. et al. (54)).

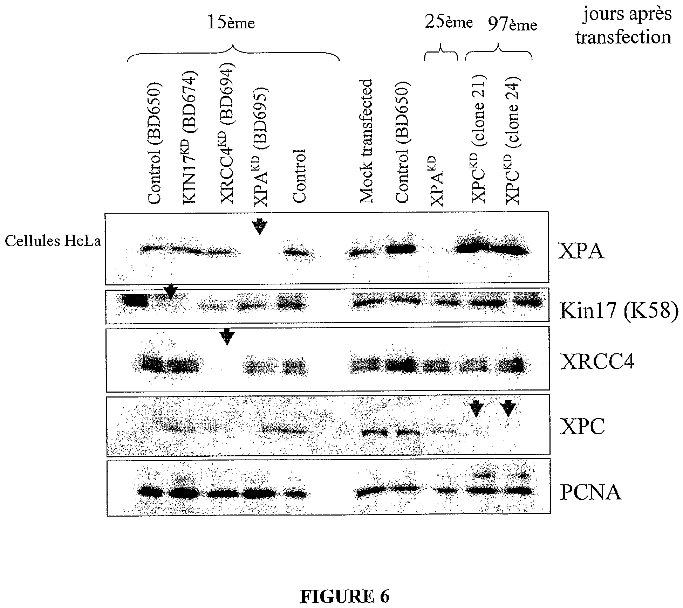

- la figure 6 illustre l**'analyse par western blot de l'extinction des gènes *XPA, XPC, XRCC4, KIN17* dans des populations stables 15 jours après la transfection ou dans des clones établis.** Après 15, 25 ou 97 jours de culture continue, les cellules sont trypsinisées, comptées et les protéines récupérées dans du tampon de lyse Laemmli 2x. L'équivalent en protéines de 100.000 cellules est analysé par western blot.

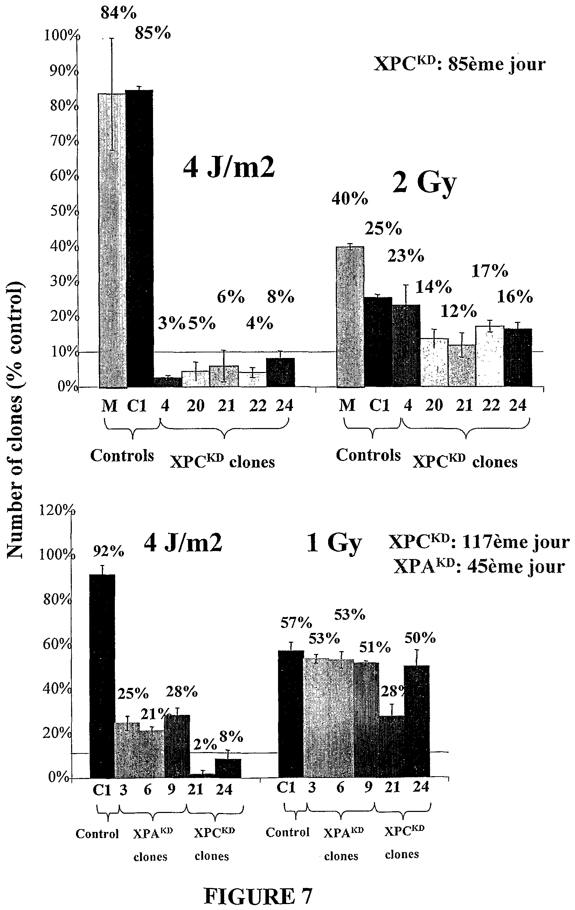

- la figure 7 illustre la **croissance clonogénique de différents clones HeLa XPA^{KD} et XPC^{KD} après irradiation UVC ou ionisante (rayons γ).** 35 cellules par cm² sont ensemencées 24h avant une irradiation. La culture est maintenue 14 jours en présence d'hygromycine B puis les clones sont fixés, colorés et comptés (seuls sont pris en compte les clones de plus de 50 cellules). Chaque point correspond à 3 boîtes de culture (±SD). Les clones XPC^{KD} présentent une plus grande sensibilité aux UVC que les clones XPA^{KD} syngéniques.

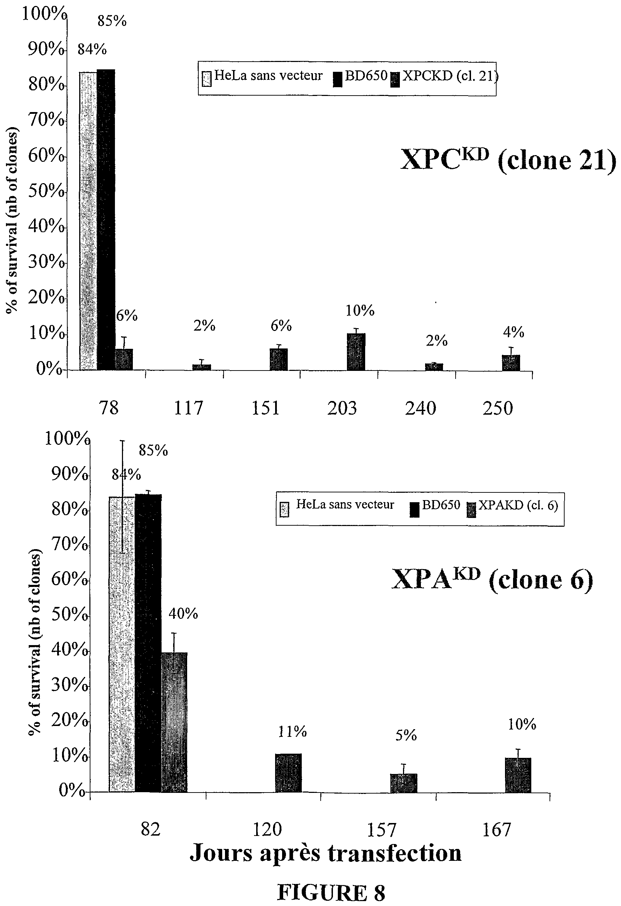

- la figure 8 illustre le **suivi de la stabilité dans le temps des clones HeLa XPA^{KD} et XPC^{KD} vis-à-vis de leur sensibilité aux rayons UVC.** A différents temps après transfection et culture continue, les cellules sont trypsinisées, mises en culture, irradiées et analysées selon les critères d'une croissance clonogénique comme décrit dans la légende de la figure 7.

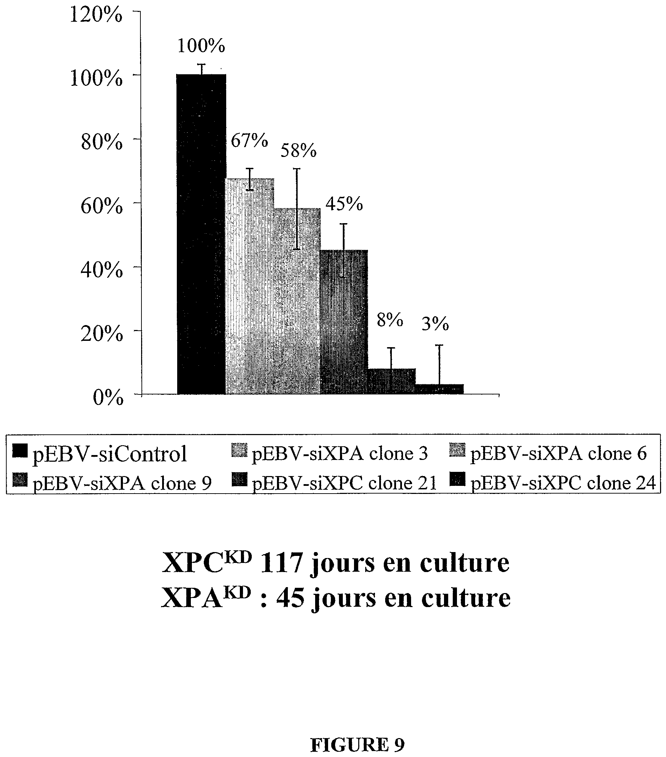

- la figure 9 illustre **la différence de l'efficacité d'ensemencement (*plating efficiency*) des clones HeLa XPC^{KD} par rapport aux clones XPA^{KD} ou aux cellules contrôles et traduit des difficultés de croissance spontanées très importantes.** A différents temps après transfection et culture continue, les cellules sont trypsinisées, mises en culture et analysées selon les critères d'une croissance clonogénique comme décrit dans la légende de la figure 7.

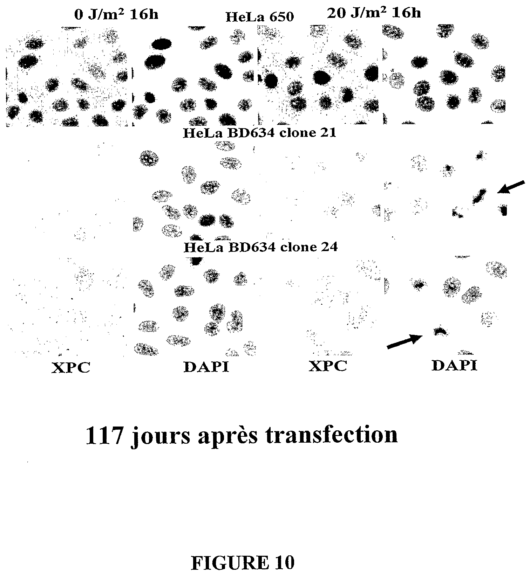

- la figure 10 illustre le fait que **la perte de l'expression du gène *XPC* est associée à une forte instabilité génomique.** Les cellules XPA^{KD}, XPC^{KD} et les cellules contrôles sont mises en culture à la même densité et irradiées à 20 J/m² (UVC). 16h après, les cellules sont fixées au paraformaldéhyde 4% (20 min) et perméabilisées au Triton x100 0.5% (5 min). Les cellules sont ensuite marquées contre XPC (ou XPA, non montré ici) et contremarquées au DAPI (marquage spécifique de l'ADN). L'extinction presque totale de l'expression du gène *XPC* est confirmée dans deux clones XPC^{KD} (clone 21 et 24). De plus, l'apparition de nombreuses figures apoptotiques est mise en évidence par le marquage DAPI (flèche sur les panneaux de droite).

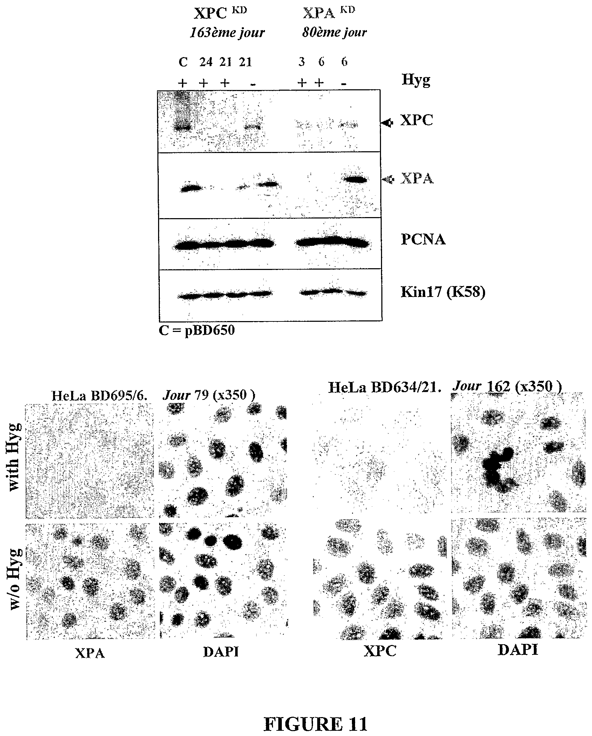

- **Figure 11** **: Démonstration de la réversibilité du système utilisé par récupération du gène rendu silencieux (*XPA* ou *XPC*).** Les clones stables depuis 163 jours (XPC^{KD}) et 80 jours (XPA^{KD}) sont mis en culture pendant 10 jours en présence ou non du marqueur de sélection M2 (hygromycine B). Les cellules sont ensuite analysées par western blot ou marquages immunocytochimiques. On constate une réapparition de l'expression des gènes *XPC* ou *XPA* dans 100% des cellules ; ce qui témoigne d'une réversion totale du phénotype qui avait été imposé pendant plusieurs mois par les vecteurs pEBV-siRNA, selon la description.

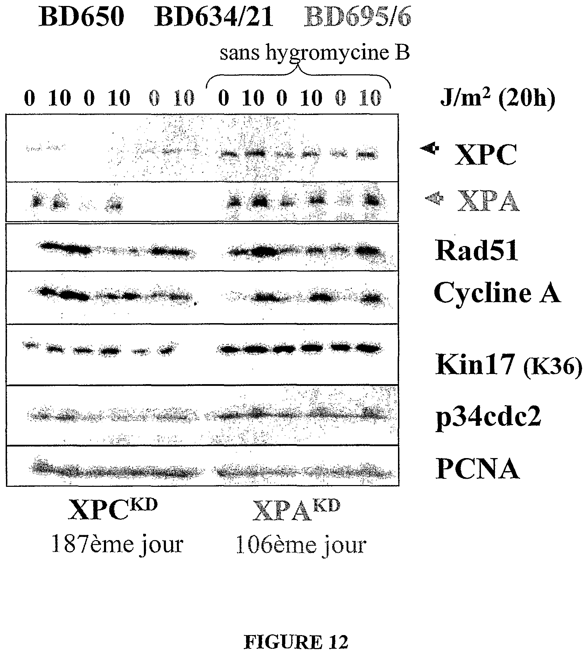

- **Figure 12** **: Nouvelle démonstration de la réversibilité du système utilisé par récupération du gène rendu silencieux (*XPA* ou *XPC*).** Dans des conditions identiques à celles de la figure 11 et dans une expérience complètement indépendante, on observe également la réversion totale du phénotype.

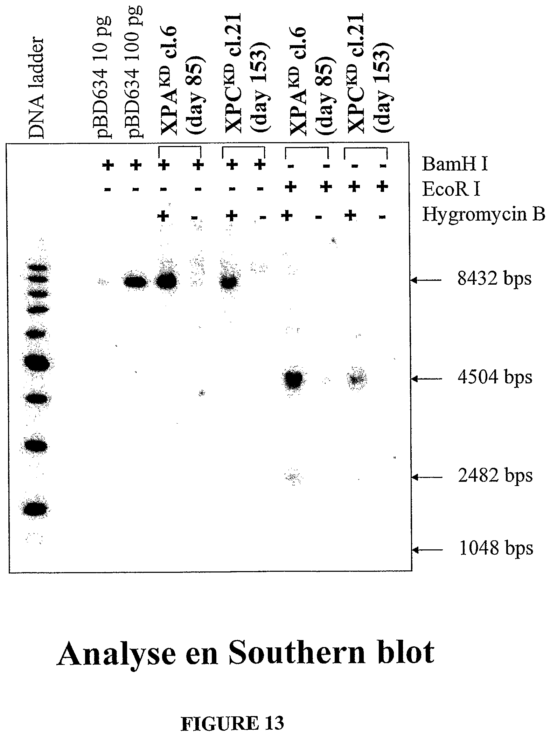

- **Figure 13** **: Perte totale des vecteurs selon la description pEBV-siRNA 10 jours après l'élimination de l'hygromycine B du milieu de culture.** Après une culture de 10 jours en présence ou non d'hygromycine B, l'ADN total (génomique + épisomal) est isolé des clones XPA^{KD} et XPC^{KD}. 10 µg est ensuite analysé par Southern blot. La sonde utilisée correspond à l'intégralité du vecteur pBD634, linéarisée et marquée au ³²P en *random priming* (Amersham). On observe les points suivants : (1) dans les clones XPC^{KD} et XPA^{KD} les vecteurs pEBV-siRNA sont maintenus sous une forme épisomale, (2) avec un nombre faible de copies par cellule (environ 10-20). (3) L'absence d'hygromycine B entraîne une perte rapide et totale de ces vecteurs.

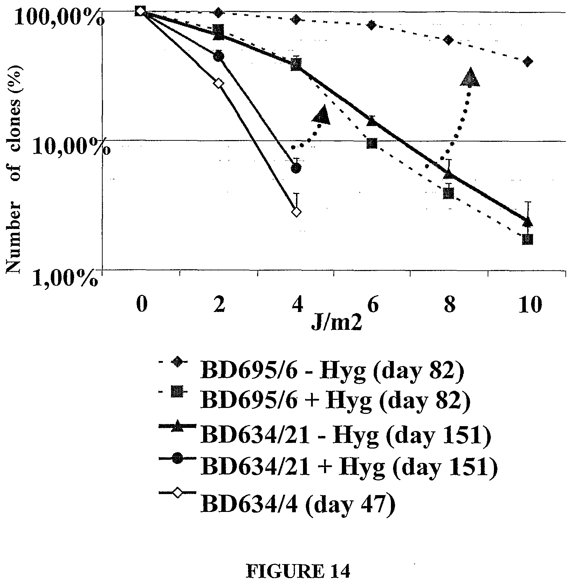

- **Figure 14** **(croissance clonogénique des cellules HeLa) : La réversion totale de l'expression des gènes XPA et XPC n'est pas toujours associée à une récupération totale d'un phénotype caractéristique (sensibilité aux UVC) : mise en évidence d'effets collatéraux de la perte d'expression du gène XPC pendant plusieurs mois.** Les cellules sont trypsinisées, mises en culture, irradiées et analysées selon les critères d'une croissance clonogénique comme décrit dans la légende de la figure 7. La culture est faite avec ou sans hygromycine B. On constate que les cellules XPA^{KD} révertées retrouvent une sensibilité normale aux UVC, alors que les cellules XPC^{KD} demeurent très sensibles aux UVC bien qu'elles aient retrouvé leur contenu normal en protéine XPC. Cela met en évidence le fait que l'absence de la protéine XPC pendant plusieurs mois a entraîné des dommages génétiques non envisageables au départ.

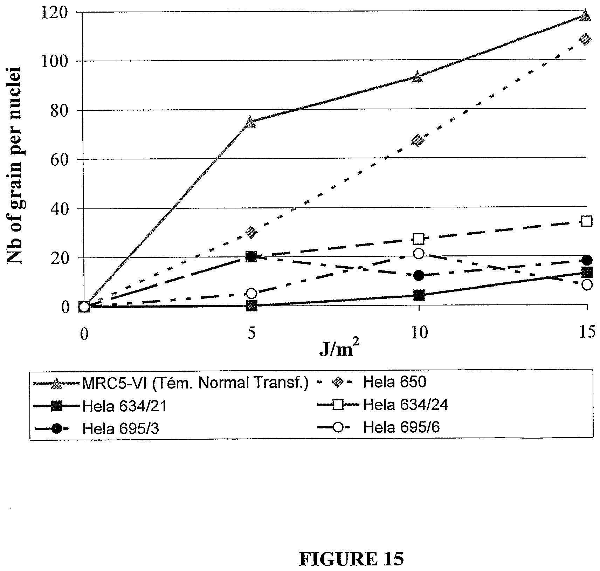

- **Figure 15** **: Défaut de réparation de l'ADN (UDS ou *unscheduled DNA synthesis*) après irradiation UVC dans les clones HeLa XPA^{KD} et XPC^{KD}.** Les cellules XPA^{KD} (clones 3 et 6) et XPC^{KD} (clones 21 et 24) sont mises en culture sur lamelle de verre et la réparation de l'ADN après irradiation UVC est suivie par l'incorporation de [³H]thymidine comme décrit précédemment (Sarasin et al. (55)).

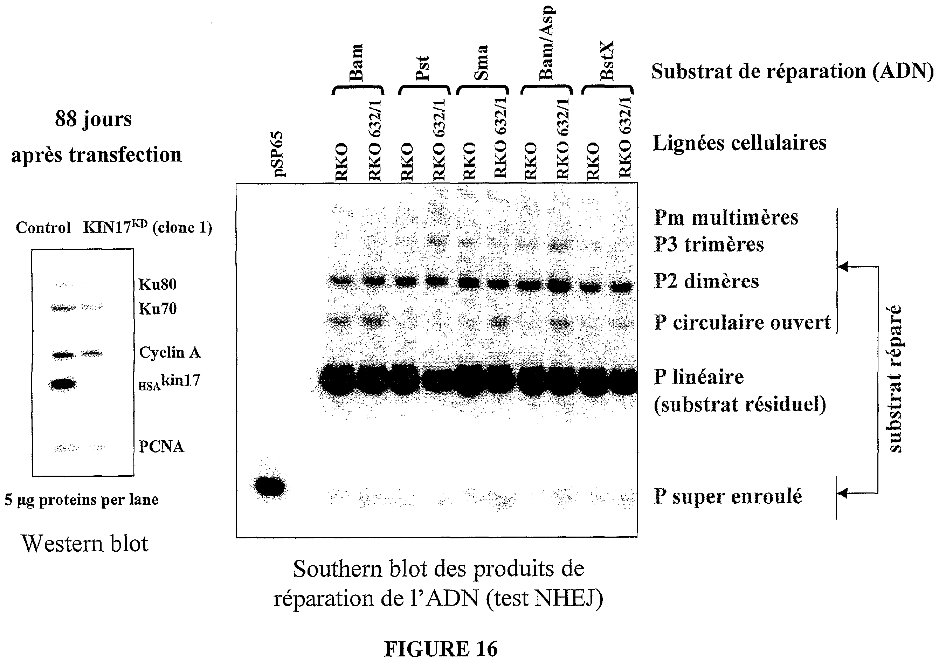

- Figure 16 : **Activité de réparation par NHEJ (*non homologous end joining*) d'un clone KIN17^{KD} en culture depuis 88 jours.** Selon un protocole adapté de Daza et al. (56), des extraits protéiques conservant une activité NHEJ sont préparés à partir d'un clone stable n'exprimant plus la protéine kin17 par l'utilisation d'un vecteur selon la description. Dans un test de réparation *in vitro,* des vecteurs plasmidiques linéarisés appelés Bam, Pst, Sma, Bam/Asp et BstX et qui présentent des extrémités franches, 5' ou 3' sortantes sont analysés par Southern blot pour constater de leur réparation (*recircularisation*). Les bandes d'ADN caractéristiques sont repérées sur la droite de la figure où « P linéaire » correspond au produit résiduel et toutes les autres bandes au produit réparé. Dans cet essai, on constate une réparation de l'ADN légèrement supérieure dans un clone RKO déficient en protéine kin17.

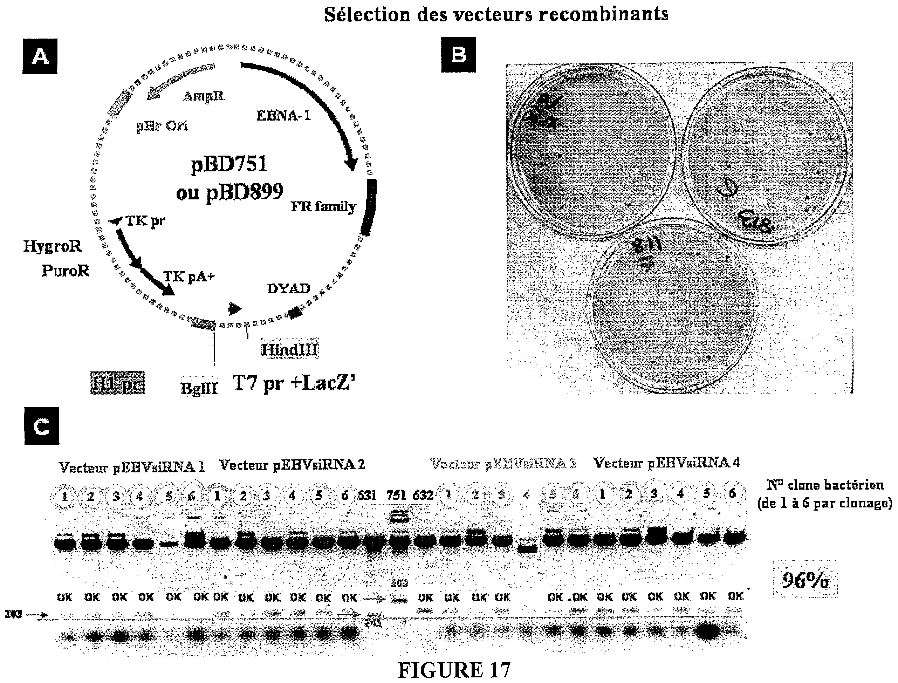

- Figure 17 : Sélection des vecteurs recombinants. (A) : représentation du vecteur de clonage (produit intermédiaire pour la préparation d'un vecteur selon la description) pBD751 (pEBV-LacZ'-hygro). (B) : boîtes de LB-agar sur lesquelles ont poussé des bactéries bleues ou blanches ; en sélectionnant les colonies blanches (6 ou 7 sont pré-sélectionnées). on sélectionne celles qui portent les vecteurs recombinants, la séquence LacZ' encadrée des sites BglII/HindIII étant remplacée par les séquences shRNA de 64 nucléotides que l'on fait synthétiser et qui sont caractéristiques d'un gène cible. (C) : analyse de quatre clonages (vecteur pEBVsiRNA 1 à 4) pour lesquels 6 clones bactériens ont été sélectionnés au hasard. Ces clones ont été amplifiés sur la journée en culture liquide (LB + Ampicilline) et leur ADN a été purifié selon les techniques classiques de la biologie moléculaire (*minipréparation d'ADN*). L'ADN de chaque clone est digéré par les enzymes de restriction HindIII / BamHI pendant 1h à 37°C. L'analyse sur gel d'agarose (1,5%) montre une efficacité de clonage proche de 100 %. L'identification des clones recombinants est faite de la manière suivante : la digestion de restriction HindIII / BamHI donne à partir du vecteur de clonage pBD751 un fragment de 509 nucléotides (correspondant aux séquences T7-LacZ') ; si le vecteur se recircularise on obtiendra un fragment de 245 nucléotides comme pour le vecteur vide pBD631 ; par contre, si l'insert (séquence shRNA) s'est correctement intégré, on aura un fragment de 303 nucléotides (comme pour le vecteur déjà cloné pBD632). Dans le cas présent, on constate que 23 des 24 clones présentent un profil de digestion parfait.

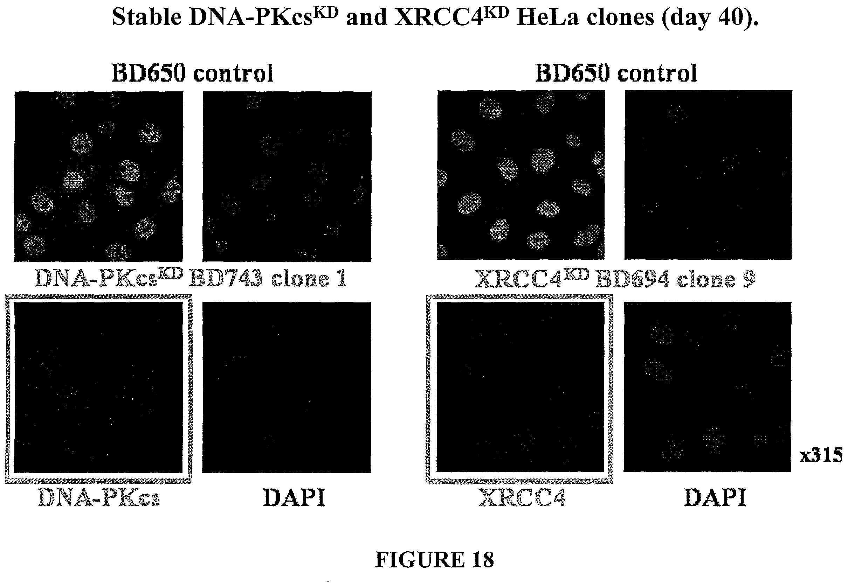

- Figure 18 : 48 h après la transfection des cellules HeLa avec soit un vecteur pBD743, soit avec un vecteur pBD694, dans les conditions exposées ci-dessus, les cellules HeLa sont ensemencées à raison de 5000 cellules/cm², en présence d'hygromycine (250 µg/ml). La détection des protéines DNA-PKcs et XRCC4 est effectuée par western blot 40 jours après la transfection (anticorps anti-DNA PKcs LabVision Neomarker #MS-423-PABX ; anticorps anti-XRCC4 Abcam clone ab145). On observe une extinction des gènes DNA-PKcs et XRCC4 : les deux vecteurs sont efficaces pour éteindre les gènes DNA-PKcs (vecteur pBD743) et XRCC4 (vecteur pBD694).

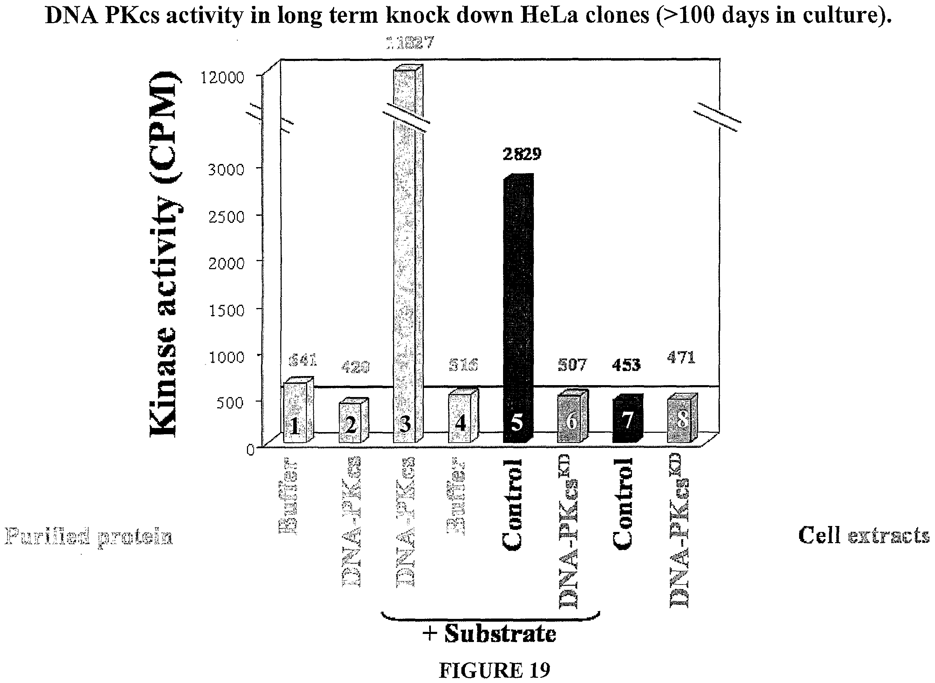

- Figure 19 : Analyse de l'activité d'un clone silencieux pour le gène DNA-PKcs (clone BD743.1 ou DNA-PKcs^{KD}, pour knock-out). Un test d'activité DNA PKcs (*pull-down assay*) a été accompli selon le protocole déjà publié par Finnie et al. (Proc. Natl. Acad. Sci. USA, 92, 320-324 1995). Brièvement, les protéines de fixation à l'ADN sont isolées sur 5 mg de résine de cellulose + ADN double chaîne (Amersham) puis l'activité DNA PKcs est testée par l'incorporation de [γ-³²P]ATP sur 4 nmol d'un substrat spécifique de la DNA PKcs (Promega). L'incorporation d'ATP radioactif sur le substrat est mesurée par scintillation liquide. Les lignes 1 à 4 et 7 et 8 correspondent aux différents contrôles.
   **(1)** Tampon seul sans aucune protéine ni substrat (c'est le bruit de fond radioactif de la technique).
   **(2)** 50 U de protéine DNA-PKcs purifiée (Promega) sans substrat.
   **(3)** 50 U de protéine DNA-PKcs purifiée (Promega) avec substrat (c'est le contrôle positif).
   **(4)** Tampon sans protéine mais avec substrat.
   **(5)** 25 µg d'extrait cellulaire d'une lignée contrôle (portant le vecteur pBD650) avec substrat (c'est le niveau basal de l'activité DNA PKcs dans des cellules HeLa).
   **(6)** 25 µg d'extrait cellulaire du clone BD743.1 (DNA PKcs knock down) avec substrat (c'est le niveau de l'activité résiduelle du clone silencieux : on est au niveau du bruit de fond de la technique).
   **(7)** 25 µg d'extrait cellulaire d'une lignée contrôle (portant le vecteur pBD650) sans substrat (contrôle négatif).
   **(8)** 25 µg d'extrait cellulaire du clone BD743.1 (DNA PKcs knock down) sans substrat (contrôle négatif).
      Après plus de 100 jours de culture, l'activité de l'enzyme est complètement absente (par rapport au bruit de fond intrinsèque à la technique mise en oeuvre).

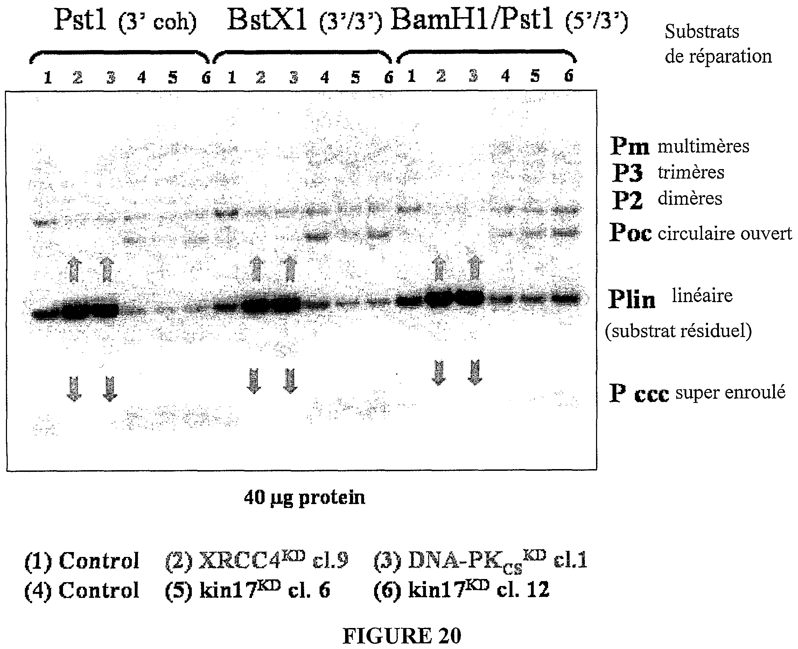

- Figure 20 : **Activité de réparation par NHEJ (*non homologous end joining*) d'un clone XRCC4^{KD} et DNA PKcs^{KD} après 75 jours de culture.** Selon le protocole décrit dans la figure 16 et adapté de Daza et al. (56), des extraits protéiques conservant une activité NHEJ sont préparés à partir de clones stables portant un vecteur selon la description. Dans un test de réparation *in vitro,* des vecteurs plasmidiques linéarisés appelés Bam, Pst, Sma, Bam/Asp et BstX et qui présentent des extrémités franches, 5' ou 3' sortantes sont analysés par Southern blot pour constater leur réparation (*recircularisation*). Les bandes d'ADN caractéristiques sont repérées sur la droite de la figure où « P lin » correspond au produit résiduel (« Produit linéaire ») et toutes les autres bandes aux produits réparés (Pccc pour « Produit circulaire super-enroulé » ; Poc pour « Produit circulaire ouvert sur un brin », P2, P3 et Pm pour les multimères). Comme le montre les flèches, on constate une réparation complètement absente dans le clone XRCC4^{KD} (clone 9) quel que soit le substrat utilisé (puits 2 pour chaque substrat) et une réparation fortement altérée pour le clone DNA PKcs^{KD} (clone 1) (puits 3 pour chaque substrat). A l'opposé, les différents autres clones silencieux KIN17^{KD} ne présentent pas de variation par rapport à la lignée contrôle portant le vecteur pBD650.
   Les figures 18-20 confirment l'intérêt des vecteurs selon la description en montrant l'extinction à long terme des protéines DNA-PKcs et XRCC4, associées à la perte de leur activité principale dans la réparation de l'ADN (recombinaison NHEJ)

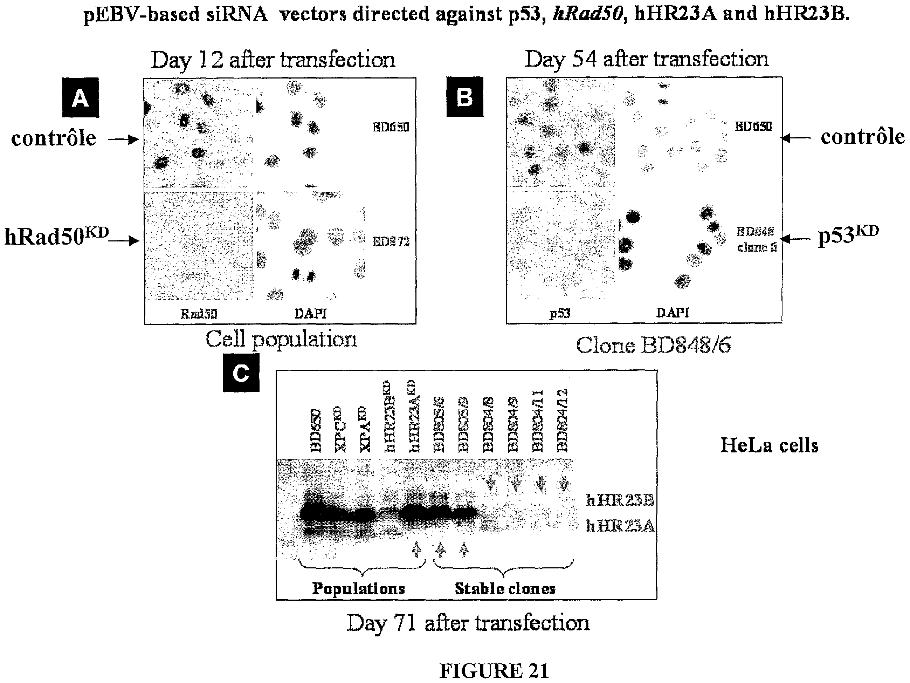

- Figure 21 : analyse des protéines par immunocytochimie (A et B) ou par western blot (C) après l'extinction des gènes hRad50, p53, hHR23A et hHR23B dans des cellules HeLa. Les cellules HeLa ont été transfectées par les vecteurs conformes à la description : vecteur pBD872, pBD848, pBD804 ou pBD805. Deux jours après la transfection, les cellules HeLa sont cultivées en présence d'hygromycine B (250 µg/ml) et analysées aux différents temps indiqués selon les protocoles décrits antérieurement (A) : Marquage immunocytochimie contre la protéine hRad50 (anticorps Calbiochem #552140). Population HeLa portant le vecteur pBD872 dirigé contre hRad50 depuis 12 jours. (B) : Marquage immunocytochimie contre la protéine p53 (anticorps DO-7 surnageant d'hybridome). Clone HeLa portant par le vecteur pBD848 dirigé contre p53 depuis 54 jours. (C) Western blot contre les protéines hHR23A et hHR23B (anticorps généreusement donnés par le Dr Kaoru Sugasawa, Ph.D, Cellular Physiology Laboratory, RIKEN Discovery Research Institute, 2-1 Hirosawa, Wako, Saitama 351-0198, JAPAN). Populations et clones cellulaires portant les vecteurs pBD804 et pBD805, respectivement dirigés contre hHR23B et hHR23A, depuis 71 jours ;

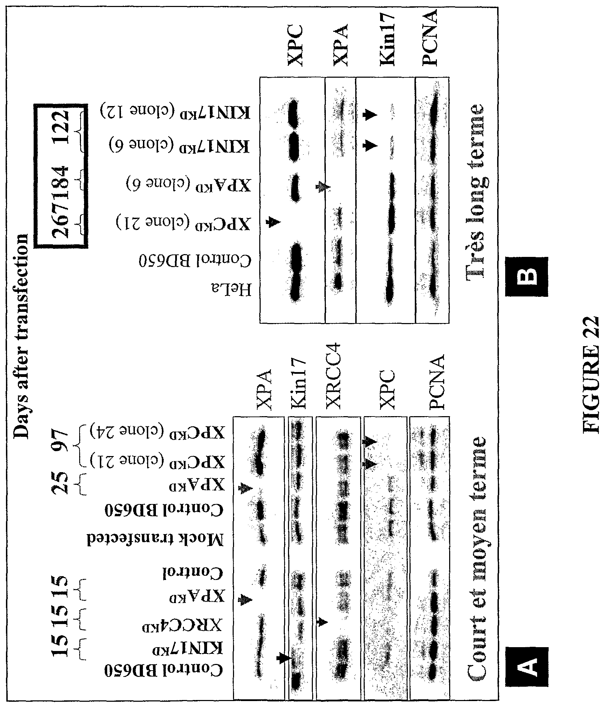

- Figure 22 : Analyse des protéines par western blot montrant l'efficacité à court et à moyen terme (A) ou à long terme (B) des vecteurs selon la description dans les cellules HeLa. Exemple de l'extinction de l'expression des gènes XPA (vecteur pBD695), XPC (pBD634), XRCC4 (pBD694), KIN17 (pBD674) après transfection de cellules HeLa et culture en présence d'hygromycine B (250 µg/ml) : pour chaque gène ciblé, de 2 à 4 vecteurs portant des séquences shRNA différentes sont construits. D'autres résultats, non représentés montrent que les clones en culture sont stables pour des périodes longues : XRCC4 : > 120 jours, DNA-PKcs : >130 jours, XPA : > 390 jours, Kin17 : > lan, hHR23B : > 150 jours, hHR23A : >150 jours, p53 : >60 jours, XPC : > 390 jours.

**Liste des séquences de la Demande**

| N° de séquences | |
|---|---|
| 1 | promoteur H1 |
| 2 | amorce sens H1 |
| 3 | amorce anti-sens H1 |
| 4 | siRNA kin17 |
| 5 | siRNA XPC |
| 6 | siRNA XPA |
| 7 | siRNA XRCC4 |
| 8 | siRNA Ku70 |
| 9 | siRNA DNA-PK |
| 10 | siRNA Rad50 |
| 11 | siRNA Rad51 |
| 12 | siRNA Rad52 |
| 13 | siRNA HR23B |
| 14 | siRNA HR23A |
| 15 | siRNA p53 |
| 16 | amorce sens pBD751 |
| 17 | amorce anti-sens pBD751 |
| 18 | oligol-siKin17R663 |
| 19 | oligo2-siKin17R663 |

Il doit être bien entendu, toutefois, que ces exemples sont donnés uniquement à titre d'illustration de l'objet de la description, dont ils ne constituent en aucune manière une limitation.

### EXEMPLE 1 : Clonage des différents vecteurs.

### - Séquences shRNA

Les séquences shRNA contiennent deux motifs de 19 nucléotides identiques dans des orientations inverses, séparées par un bras espaceur de 9 pb de séquences non homologues (TTCAAGAGA ou toute autre séquence). En 5' de cet oligonucléotide « sens » est introduit un site de clonage pour l'enzyme de restriction Bgl II (ou autre) suivi d'une séquence de départ de la transcription (généralement CCG). En 5' de l'oligonucléotide « antisens » est introduit un fragment de 6 thymidines qui constitue un signal de terminaison pour l'ARN Pol III. Un site de clonage Hind III (ou autre) est introduit à l'extrémité 3' des séquences shRNA.

La figure 1 illustre une telle construction pour un siRNA conçu contre l'ARNm du gène *KIN17* et correspond aux positions 663-681 (siK663/kin17), en référence à la séquence accessible sous le numéro d'accès Genbank NM-012311.

### - Stratégie de clonage

La stratégie de clonage avec un oligodésoxynucléotide (Eurogentec, Belgique) est également illustrée à la figure 1A. Les oligodésoxynucléotides sens et anti-sens sont hybridés de manière à former un ADN double-brin avec des extrémités protrusives Bgl II et Hind III de la façon suivante : les oligodésoxynucléotides sont repris dans de l'eau stérile autoclavée (sans nucléase) à la concentration de 3 mg/ml. 1 µl de chaque oligodésoxynucléotide (à 3 mg/ml) est prélevé et 48 µl de tampon (NaCl 100 mM ; Tris pH 7,5 50 mM) sont ajoutés. L'appariement est fait dans un thermocycleur (appareil à PCR de marque indéterminé) selon le programme suivant : 95°C pendant 5 min, puis 5 min aux températures suivantes : 90°C, 85°C, 80°C, 75°C et 70°C. Cela est suivi d'un maintien à 37°C pendant 30 min et à 4°C jusqu'à la récupération des échantillons. Les oligodésoxynucléotides appariés sont conservés ensuite à -20°C jusqu'au clonage.

### - Promoteur H1 RNA

Pour cloner le promoteur H1-RNA dans le vecteur pSUPER, les amorces PCR suivantes (Brummelkamp et al. 2002 (5)) ont été utilisées :
Sens : 5'-CCATGGAATTCGAACGCTGACGTC-3' (SEQ ID NO :2) et
Antisens : 5'-GCAAGCTTAGATCTGTGGTCTCATACAGAACTTATAAGATT CCC-3' (SEQ ID NO:3),

Un clonage direct des oligonucléotides appariés (codant un shRNA) est obtenu en utilisant les vecteurs pBD751 (pEBVsiR-LacZ') ou pBD899 (pEBVsiR-LacZ'-Puro) comme décrit dans la figure 1C. Les vecteurs pBD631 (pEBVsiR), pBD884 (pEBVsiR-Puro) ou pBD665 (pEBVsiD ; vecteur portant la cassette siRNA dans le sens opposé à celui du vecteur pBD631) peuvent aussi être utilisés.

De même un clonage indirect peut être entrepris après introduction des oligonucléotides appariés dans le vecteur pSUPER puis récupération de la cassette siRNA (H1 promoteur + shRNA) par digestion Kpn I / BamH I (New England Biolabs). Cette cassette siRNA est ensuite introduite dans un vecteur EBV comme le vecteur pBD149 (10).

Dans l'un ou l'autre cas, les cassettes siRNA sont vérifiées par séquençage.

La présence des cassettes siRNA (promoteur H1 + séquence codant pour un shRNA) est vérifiée par séquençage, récupérée après digestion par les enzymes Kpn I / BamH I (New England Biolabs) et introduit dans un vecteur pBD149 (10). Un clonage direct dans les vecteurs pEBV portant le promoteur H1 sans séquence shRNA est aussi efficace selon la même stratégie, à savoir une digestion Bgl II / Hind III du vecteur pBD631 (pBD665, qui porte la cassette siRNA dans le sens opposé à celui du vecteur pBD631 ou du vecteur pBD884 ; voir Tableau I) suivi de l'insertion des oligodésoxynucléotides appariés. Les vecteurs EBV pBD631 et pBD665 portent le promoteur H1 et les sites de clonage Bgl II / Hind III dans les deux orientations possibles. Ainsi, les clonages peuvent se faire directement dans ces vecteurs pBD631 et pBD665 sans passer par le vecteur pSUPER.

### - Construction de vecteurs intégratifs

Des plasmides intégratifs selon l'Art antérieur sont également construits après délétion des séquences EBV du plasmide précédent (digestion Hpa I / BstE II, remplissage Klenow et auto-ligation). Le remplissage Klenow permettant d'obtenir des extrémités franches (« *blunt ends* »), est effectué selon le protocole suivant : après digestion, des vecteurs pEBV-siRNA avec les enzymes de restriction Hpa I puis BstE II (New England Biolabs, selon les recommandations du fournisseur), 1 mM de chaque dNTP (50 µM final pour chacun) et 1 unité d'enzyme Klenow (fragment Klenow 3'→ 5' exo- ; 5 U / µL) sont ajoutés. Les échantillons sont incubés à température ambiante pendant 15 min et la réaction est arrêtée par chauffage à 75°C pendant 10 min.

Les vecteurs utilisés sont répertoriés dans le Tableau I ci-après.

**TABLEAU I**

| **Gènes ciblés** | **Vecteurs pEBV réverse** | **Résistance** | **SiRNA*** | **Vecteurs pEBV direct** | **Vecteurs pHygro** | **Vecteurs pSUPER** |
|---|---|---|---|---|---|---|
| Vecteur vide | pBD631 (pEBVsiR) | Hygromycine B | - | pBD665 (pEBVsiD) | pBD641 (pHygrosi) | pBD621 (pSUPERsi) |
| | pBD884 (pEBVsiR-Puro) | Puromycine | | | | |
| Vecteur de clonage | pBD751 (pEBVsiR-LacZ') | Hygromycine B | LacZ' pour clonage | | | |
| | pBD899 (pEBVsiR-LacZ'-Puro) | Puromycine | | | | |
| Vecteur contrôle | pBD650 (pEBVsiControlR) | Hygromycine B | Contrôle (shRNA muté) | | pBD653 (pHygrosiControl) | pBD647 (pSUPERsiControl) |
| | pBD886 (pEBVsiControlR-Puro) | Puromycine | | | | |
| KIN17 | pBD674 (pEBVsiK180R) | Hygromycine B | siK180 | pBD678 (pEBVsiK180D) | | pBD667 (pSUPERsiK180) |
| | pBD901 (pEBVsiK180R-Puro) | Puromycine | 180-198 | | | |
| KIN17 | pBD685 (pEBVsiK406R) | Hygromycine B | siK406 | pBD686 (pEBVsiK406D) | | pBD645 (pSUPERsiK406) |
| | | Puromycine | 406-424 | | | |
| KIN17 | pBD632 (pEBVsiK663R) | Hygromycine B | siK663 | pBD664 (pEBVsiK663D) | pBD642 (pHygrosiK663) | pBD622 (pSUPERsiK663) |
| | pBD900 (pEBVsiK663R-Puro) | Puromycine | 663-681 | | | |
| KIN17 | pBD676 (pEBVsiK906R) | Hygromycine B | siK906 | pBD680 (pEBVsiK906D) | | pBD668 (pSUPERsiK906) |
| | | | 906-924 | | | |
| XPC | pBD634 (pEBVsiXPC-267R) | Hygromycine B | siXPC-267 | | pBD657 (pHygrosiXPC267) | pBD624 (pSUPERsiXPC267) |
| | pBD894 (pEBVsiXPC-267R-Puro) | Puromycine | 267-285 | | | |
| XPA | pBD695 (pEBVsiXPA-587R | Hygromycine B | siXPA-587 | | | pBD688 (pSUPERsiXPA587) |
| | pBD895 (pEBVsiXPA-587R-Puro) | Puromycine | 587-605 | | | |
| XRCC4 | pBD694 (pEBVsiXRCC4-674R) | Hygromycine B | siXRCC4-674 | | | |
| | pBD902 (pEBVsiXRCC4-674R-Puro) | Puromycine | 674-692 | | | |
| Ku70 | pBD699 (pEBVsiKu70-156R) | Hygromycine B | siKu70-156 | | | |
| | pBD903 (pEBV-siKu70-156R-Puro) | Puromycine | 156-174 | | | |
| DNA-PKcs | pBD743 (pEBVsiDNAPK-5980R) | Hygromycine B | siDNAPK-5980 | | | |
| | pBD904 (pEBV-siDNAPK-5980R-Puro) | Purmocyine | 5980-5998 | | | |
| Rad50 | pBD872 (pEBVsiRad50-274R) | Hygromycine B | Rad50-274 | | | |
| | pBD908 (pEBVsiRad50-274R-Puro) | Puromycine | 274-292 | | | |
| Rad51 | pBD864 (pEBVsiRad51-114R) | Hygromycine B | siRad51-114 | | | |
| | pBD917 (pEBVsiRad51-114R-Puro) | Puromycine | 114-132 | | | |
| Rad52 | pBD876 (pEBVsiRad52-502R) | Hygromycine B | Rad 52-502 | | | |
| | pBD910 (pEBVsiRad52-502R-Puro) | Puromycine | 502-520 | | | |
| hHR23B | pBD804 (pEBVsiR23B-1123R) | Hygromycine B | siR23B-1123 | | | |
| | pBD896 (pEBVsiR23B-1123R-Puro) | Puromycine | 1123-1141 | | | |
| hHR23A | pBD805 (pEBVsiR23A-491R) | Hygromycine B | siR23A-491 | | | |
| | pBD897 (pEBVsiR23A-491R-Puro) | Puromycine | 491-509 | | | |
| p53 | pBD848 (pEBVsip53-112R) | Hygromycine B | sip53-112 | | | |
| | pBD906 (pEBVsip53-112R-Puro) | Puromycine | 112-130 | | | |
| hTERT | pBD839 (pEBVsihTERT-1730R) | Hygromycine B | sihTERT-1730 | | | |
| | pBD905 (pEBVsihTERT-1730R-Puro) | Puromycine | 1730-1748 | | | |
| SAF-A | pBD775 (pEBVsiSAFA-1955R) | Hygromycine B | siSAFA-1955 | | | |
| | pBD907 (pEBVsiSAFA-1955R-Puro) | Puromycine | 1955-1973 | | | |

| | | | | | | |
|---|---|---|---|---|---|---|
| * les séquences sont précisées dans le Tableau II. | | | | | | |

Des plasmides contrôles sont également construits :
- des plasmides qui portent un promoteur H1 sans siRNA (pBD631 et pBD665 précités),
- des plasmides qui portent un promoteur H1 et un système de clonage LacZ' sans siRNA (pBD751 et pBD899 précités),
- un plasmide obtenu après insertion d'un shRNA portant deux misappariements sur l'un des brins de la structure en épingle à cheveux (pBD650).

L'ADN plasmidique est purifié avec une résine échangeuse d'anions (Qiagen) et transfecté.

Les différents siRNAs pour XPA, XPC, _{HSA}kin17, XRCC4, Ku70, DNAPK, Rad50, Rad51, Rad52, hHR23B, hHR23A et p53 sont construits selon les règles reconnues pour les siRNA (2, 3, 16-18).

Les séquences d'ADN synthétiques codant les séquences shRNA sont adaptées du modèle décrit par Brummelkamp et al. (5) avec CCG en 5' comme site d'initiation de la transcription et 6 thymidines en 3' comme signal de terminaison et sont introduites dans le vecteur vide selon la description comme précisé ci-dessus dans les deux orientations possibles afin de vérifier le rôle de leur position par rapport aux autres éléments présents dans ledit vecteur.

Dans le Tableau I, le nom de chaque séquence siRNA correspond à :
si : siRNA
K : _{HSA}kin17
XPA : XPA
XPC : XPC
nombre : position du 1^{er} nucléotide dans la séquence siRNA par rapport à l'ADNc correspondant : par exemple pour KIN17 : « siK180 » signifie une séquence siRNA contre l'ARNm *KIN17* reconnaissant comme premier nucléotide le 180^{ème} de la séquence de l'ADNc *KIN17* (et les 18 autres suivants).

Les siRNA ont été conçus conformément aux recommandations figurant dans la littérature. En particulier, les constructions prennent en compte ou ne prennent pas en compte les caractéristiques thermodynamiques des séquences siRNA (2, 3, 16, 17).

Le Tableau II précise certaines des séquences siRNA mises en oeuvre.

**TABLEAU II**

| Séquence du siRNA | Gène |
|---|---|
| Position du siRNA dans l'ADNc | |
| TCCTCAGCAGTTTATGGAT (SEQ ID NO :4) | Kin17 |
| 180-198 | |
| GGATGAAGCCCTCAGCGAT (SEQ ID NO :5) | XPC |
| 267-285 | |
| GTCAAGAAGCATTAGAAGA (SEQ ID NO :6) | XPA |
| 587-605 | |
| GAGATCCAGTCTATGATGA (SEQ ID NO :7) | XRCC4 |
| 674-692 | |
| GAGTGAAGATGAGTTGACA (SEQ ID NO :8) | Ku70 |
| 156-174 | |
| GTTGAGGTTCCTATGGAAA (SEQ ID NO :9) | DNA-PK |
| 5980-5998 | |
| GAACTTATAGCTGTGCAAA (SEQ ID NO : 10) | Rad50 |
| 274-292 | |
| GAAGAAATTGGAAGAAGCT (SEQ ID NO : 11) | Rad51 |
| 114-132 | |
| GACAAAGACTACCTGAGAT (SEQ ID NO : 12) | Rad52 |
| 502-520 | |
| GCATTAGGATTTCCTGAAG (SEQ ID NO : 13) | HR23B |
| 1123-1141 | |
| AGACGATGCTGACGGAGAT (SEQ ID NO : 14) | HR23A |
| 491-509 | |
| CAAGCAATGGATGATTTGA (SEQ ID NO : 15) | p53 |
| 112-130 | |

### EXEMPLE 2 : Efficacité comparée des duplex siRNA, des plasmides intégratifs et des vecteurs comprenant un segment EBV.

### - Inhibition de l'expression du gène KIN17

Pour pouvoir comparer l'efficacité de l'inhibition de l'expression d'un gène, par différents types de constructions, des duplex siRNA (siK180 et siK906) et les séquences ADN codant les shRNA associés ont été synthétisés, respectivement par Ambion pour les siRNA et Eurogentec ou Proligo pour les ADN. Les séquences ADN codant les shRNA ont été introduites comme décrit précédemment dans des vecteurs EBV donnant les vecteurs pBD674 (ou pBD678) et pBD676 (ou pBD680) selon l'orientation de la cassette siRNA. Cette approche permet de comparer l'efficacité des vecteurs pEBV-siRNA par rapport aux duplex siRNA dont ils dérivent, après transfection dans des cellules HeLa ou RKO, dans les conditions précisées dans la légende de la figure 2.

Trois jours après la transfection, on observe une diminution du taux de protéine _{HSA}kin17 de l'ordre de 80-90 % sans modification des autres protéines nucléaires testées (figure 2), ce qui démontre l'efficacité des vecteurs pEBV-siRNA selon la description, qui conduisent à une extinction de l'expression du gène *KIN17* similaire à celle observée après transfection des duplex siRNA. Cela témoigne aussi de la spécificité de la répression car le niveau des autres protéines contrôle n'est pas modifié.

Le maintien dans le temps et la stabilité des vecteurs pEBV (en présence d'hygromycine B) améliorent considérablement l'inhibition de l'expression des gènes testés, comme ici le gène *KIN17* (figure 3). Il en est de même pour les gènes XPA ou XPC (voir plus loin).

L'orientation de la cassette siRNA dans les vecteurs n'influence pas le taux d'inhibition de l'expression.

La délétion du segment EBV dans les vecteurs conduit à l'obtention de plasmides intégratifs (pHygro) du type de ceux décrits dans la littérature (voir 5).

L'efficacité des vecteurs pEBV-siRNA, selon la description, en comparaison avec les vecteurs existant de type intégratif (pHygro-siRNA), peut être démontrée selon deux critères : (1) le nombre de clones sélectionnés 14 jours après transfection dans des conditions de culture clonogénique (peu de cellules mises en culture en présence de l'hygromycine B), et (2) le nombre de cellules de ces clones rendues efficacement silencieuses pour l'expression d'un gène donné (par exemple : *KIN17*)*.* Les résultats obtenus sont illustrés à la figure 4 et dans le Tableau III ci après.

| **Vecteur** | **Nombre de clones ± SD par 10⁶ cellules ensemencées** | |
|---|---|---|
| | **cellules HeLa** | **cellules RKO** |
| **pBD631** | | |
| pEBV-siR | 44250 ± 2136 | 42980 ± 2608 |
| | | |
| **pBD632** | | |
| pEBV-siK663R | 417 ± 144 | 37475 ± 1919 |
| | | |
| **pBD650** | | |
| pEBV-siControlR | 50833 ± 5700 | 46667 ± 5122 |
| | | |
| **pBD641** | | |
| pHygro-siR | 194 ± 48 | 2205 ± 287 |
| | | |
| **pBD642** | | |
| pHygro-siK663R | 56 ± 48 | 2205 ± 278 |
| | | |
| **pBD653** | | |
| pHygro-siControlR | 28 ± 48 | 17 ± 29 |

48 h après la transfection, 150 cellules (transfectées par les vecteurs selon la description) ou 500 cellules (transfectées par des vecteurs pHygro)/cm² (soit 5 000 cellules transfectées par un vecteur pEBV selon la description et 15 000 cellules transfectées par un vecteur pHygro) sont ensemencées en présence d'hygromycine B. Quatorze jours après, les clones sont fixés, colorés et comptés. Chaque point correspond à la moyenne de trois boîtes de culture. En présence d'un vecteur intégratif (pHygro) similaire à ceux rencontrés dans la littérature, on observe peu de clones en croissance après 14 jours et plus de 50 % de ces clones n'inhibent pas l'expression du gène *KIN17* (figure 4.2).

Au contraire, on observe de nombreux clones après transfection avec des vecteurs selon la description, tous ces derniers inhibant l'expression du gène *KIN17* (figure 4.4).

### - XPA et XPC

L'ensemble des résultats correspond à une compilation de plus de 10 essais indépendants, témoignant de la reproductibilité de ces expériences, la plupart des expériences ayant été menées dans les cellules HeLa, et secondairement MRC5-V2 et RKO.

Des essais similaires à ceux conduits avec *KIN17* ont été entrepris avec les gènes *XPA* et *XPC* dans les cellules HeLa, MRC5-V2 et RKO. Les principaux résultats obtenus sont illustrés dans les figures 5 à 10 et ils démontrent l'efficacité remarquable des vecteurs selon la description pour éteindre durablement et spécifiquement l'expression d'un gène et ce, même après plus de 260 jours de culture.

L'efficacité des vecteurs selon la description est mise en évidence lors de l'analyse aléatoire de clones (ici 12 clones choisis au hasard et éteints pour l'expression du gène *XPA*) où l'on peut obtenir jusqu'à 100% de clones éteints 48 jours après la transfection (culture en présence d'hygromycine B). Un tel résultat n'est jamais obtenu avec des vecteurs intégratifs (type pHygro). Dans ces expériences, les clones XPC^{KD} (maintenus pendant 113 jours en culture continue) servent de contrôle. On observe par ailleurs que le niveau des autres protéines analysées ne change pas, témoignant de la spécificité de l'extinction.

### - Vérification de la spécificité et de la stabilité dans le temps

La figure 6 illustre les résultats de deux expériences simultanées, l'une analysée à moyen terme (après 15 jours de culture continue) et la seconde à long et très long terme (25 et 97 jours de culture). Dans les deux cas, on observe la très forte efficacité et spécificité des constructions selon la description, avec une réduction supérieure à 95% du niveau des protéines cibles comme XPA, kin17, XRCC4 ou encore XPC (essais croisés).

Ces résultats montrent que la construction selon la description fonctionne quelle que soit la séquence shRNA sélectionnée.

De façon particulièrement intéressante, 15 jours après la transfection (côté gauche de la figure 6), il s'agit de l'analyse de populations cellulaires (et non de clones sélectionnés comme ce qui est montré sur le côté droite de cette même figure). Cela démontre l'efficacité des vecteurs selon la description pour réduire globalement et dans toutes les cellules d'une population (par définition hétérogène) le niveau d'expression du gène cible.

Les clones sélectionnés et maintenus en culture, sont régulièrement analysés pour leur principal phénotype associé à la perte de l'expression des protéines XPA et XPC, à savoir leur hypersensibilité aux UVC. La figure 7 démontre cela avec plusieurs clones XPA^{KD} ou XPC^{KD}. A l'opposé, peu ou pas de sensibilité particulière n'est observée après une irradiation ionisante (rayons y).

En outre, pour vérifier qu'il n'existe pas d'effet non spécifique, des vecteurs vides portant le promoteur H1 dans les deux orientations différentes sans séquence siRNA (pBD631 et pBD665) et un vecteur pEBV (pBD650) contenant un oligodésoxynucléotide codant pour un shRNA portant deux misappariements dans l'un des brins de la structure en épingle à cheveux ont également été testés.

Ces différents vecteurs ne modifient pas l'expression du gène (figures 2-5).

### - Stabilité des cellules XPA^{KD} et XPC^{KD}

Comme énoncé précédemment, la stabilité des clones maintenus en culture est analysée régulièrement par rapport à leur sensibilité aux UVC. La figure 8 démontre l'hypersensibilité dans le temps des clones, même après 250 jours de culture (clone XPC^{KD}). Il est à remarquer que dans la littérature, aucun clone n'a atteint une telle stabilité dans le temps.

### - Conclusions

Les vecteurs selon la description induisent une extinction des gènes étudiés dans plus de 95 % des cellules, deux semaines après la transfection, alors que des vecteurs intégratifs (pHygro-siRNA) induisent moins de 50 % de cellules éteintes après deux semaines.

La plupart des clones isolés n'expriment pas le gène cible (100 % pour XPA).

Plus de 170 jours après la transfection, les clones sélectionnés continuent à exprimer très faiblement le gène cible.

Le nouveau phénotype associé à la perte de la protéine cible est ainsi maintenu.

Tous les résultats sont reproductibles dans différentes lignées cellulaires (HeLa, RKO, MRC5-V2) avec différents vecteurs selon la description et quel que soit le shRNA (siRNA) sélectionné.

### EXEMPLE 3 : Inhibition de l'expression des gènes XPA et XPC à long terme dans des cellules humaines.

Les vecteurs pEBV selon la description permettent d'obtenir des inhibitions d'expression à long terme (voir figures 5 et 6 par exemple).

Ceci peut s'expliquer par les caractéristiques des plasmides sélectionnés qui persistent dans les noyaux comme épisomes stables après une longue période de mise en culture.

La réplication de l'ADN de ces vecteurs est semi-conservative, s'initie autour de la dyade (DS) et est coordonnée à la réplication génomique.

Des facteurs cellulaires de l'hôte assistent la réplication EBV. En particulier, Orc2 interagit étroitement avec la séquence EBNA-1 et restreint la réplication EBV à un seul tour par cycle cellulaire, ce qui explique qu'il y ait peu de copies du vecteur/cellule et une stabilité importante.

La présence d'EBNA-1 lie aussi les épisomes EBV à la métaphase, ce qui est un facteur de leur rétention nucléaire et de leur ségrégation lors de la mitose.

En outre, les vecteurs EBV selon la description peuvent se comporter comme des unités de transcription endogènes contrôlant étroitement la transcription des siRNA.

Avec ces vecteurs, il est possible d'isoler des clones présentant des taux d'expression indétectables des gènes XPA et XPC (figures 5 et 6).

Ces différents clones XPA^{KD} et XPC^{KD} ont pu d'ores et déjà être maintenus en culture plus de plus de 260 jours pour les clones XPC^{KD} et 170 jours pour les clones XPA^{KD}.

Dans les cellules MRC5-V2, des clones XPA^{KD}, XPC^{KD} et KIN17^{KD} sont également en culture depuis plus de 60 jours.

La sensibilité accrue aux UV des cellules XPA^{KD} et XPC^{KD} illustre l'efficacité fonctionnelle des vecteurs selon la description.

Il est ainsi possible de comparer facilement les phénotypes des cellules HeLA, HeLa XPA^{KD} et HeLa XPC^{KD}. En effet, ces cellules possèdent toutes la même information génétique et différent seulement par la petite séquence shRNA codée par le vecteur selon la description et peuvent être considérées comme étant des lignées presque isogéniques (ou quasi isogéniques).

Tandis que l'inhibition du gène XPC, dans les cellules HeLa, induit une sensibilité aux UVC (19 fois plus sensibles à 4 J/m² que les cellules contrôle), les cellules incluant des vecteurs siRNA contre le gène *XPA* présentent une plus faible augmentation de la sensibilité aux UV (4 fois).

Les résultats sont illustrés à la figure 7.

Les cellules XPA^{KD} et XPC^{KD} ne présentent pas de sensibilité significative aux radiations ionisantes.

Par contre, les cellules XPA^{KD} ont une croissance relativement rapide par rapport aux cellules XPC^{KD} dans les cellules HeLa (figure 9) et dans d'autres cellules (MRC5-V2).

Ceci suggère que la perte partielle de XPA n'affecte pas de manière significative la physiologie cellulaire.

Par contre, l'inhibition du gène XPC entraîne des anomalies dans la croissance des cellules ainsi modifiées et seulement quelques clones émergent des cultures.

Par contre, une fois établis, ces derniers clones deviennent remarquablement stables.

### - Etude de l'effet de l'inhibition

Les cellules XPC^{KD} présentent des micronoyaux spontanés et induits par les UVC ainsi que de nombreuses cellules apoptotiques (comme le montre les flèches, sur la figure 10) après irradiation UVC, indiquant une augmentation de l'instabilité génomique.

Ces résultats sont illustrés au Tableau IV ci-après ainsi qu'à la figure 10.

**TABLEAU IV**

| | **0 J/m²** | **20 J/m²** |
|---|---|---|
| Contrôle | 3 % | 14 % |
| BD695/3 (XPA^{KD}) | 3 % | 39 % |
| BD634/21 (XPC^{KD}) | 10 % | 69 % |
| BD634/24 (XPC^{KD}) | 13 % | 59 % |

| | | |
|---|---|---|
| XPA^{KD} : 148 jours en culture XPC^{KD} : 77 jours en culture. | | |

### EXEMPLE 4: Étude de l'effet de la pression de sélection : intérêt du modèle selon la description

Les cellules transfectées avec les plasmides selon la description sont soumises à la pression de sélection (voir exemple 2, Tableau III).

Les vecteurs pEBV-siRNA selon la description se maintiennent sous une forme épisomale (non intégrée à l'ADN génomique) pendant de très longs mois. Ainsi, l'élimination de l'hygromycine B du milieu de culture doit permettre aux cellules de perdre ce vecteur et de récupérer l'expression du gène précédemment éteint (par exemple, *XPC* ou *XPA*). Il est à remarquer que cela est strictement impossible dans le cas de vecteurs classiques de type intégratif où il y a une insertion dans l'ADN génomique de très nombreuses copies de ces vecteurs.

Dans le cas des vecteurs selon la description et comme le démontre les différentes expériences réalisées, dès l'élimination de l'hygromycine B, la réversion survient très rapidement (en moyenne 7 à 10 jours) comme en témoignent les marquages en immunocytochimie où 95-100% des cellules ré-expriment les protéines XPC ou XPA seulement après 7 jours de culture en absence d'hygromycine B. Cela met en évidence une perte des vecteurs EBV-siRNA simultanément dans toutes les cellules en culture. Ce résultat est vérifié par l'analyse de l'ADN total (génomique + épisomal) par Southern blot (Figure 13). La figure 13 démontre les points suivants :
(1) dans les clones XPC^{KD} et XPA^{KD} les vecteurs pEBV-siRNA sont bien maintenus sous une forme épisomale,
(2) avec un nombre faible de copies par cellule (environ 10),
(3) l'absence d'hygromycine B pendant 10 jours entraîne la perte rapide et totale de ces vecteurs, ce qui explique la récupération très rapide de l'expression des gènes *XPC* ou *XPA* observé en immunocytochimie.

Cette réversion totale et rapide permet de démontrer que la perte de l'expression d'un gène donné peut induire des effets collatéraux inattendus.

Pour la première fois, c'est un moyen de tester les conséquences parallèles et non envisageables au départ, des effets d'une séquence siRNA. Il ne s'agit pas de mettre en évidence des cibles non-spécifiques (*off targets*) déjà déterminées par ailleurs, mais de déterminer les effets réels, spécifiques et collatéraux de la perte de l'expression d'un gène donné. En pratique, cela est mis en évidence en mettant en oeuvre les vecteurs selon la description et plus particulièrement pour l'extinction du gène XPC où la réversion ne permet pas de récupérer l'intégralité du phénotype de départ.

Cette propriété peut notamment être utilisée pour restaurer le phénotype de départ dans le cadre d'une étude fine du criblage des siRNA et mieux préciser les effets secondaires éventuels de l'inhibition de l'expression du gène ciblé, à long terme.

### EXEMPLE 5 : Analyse de l'activité de complexes multi-protéiques actifs in vitro dans des clones (ou populations) isogéniques dérivant seulement par la perte (presque totale) de l'expression d'un gène essentiel : utilisation d'un modèle cellulaire conforme à la description (cellules modifiées par un vecteur convenable selon la description)

L'obtention de clones très stables après plusieurs mois de culture, et même après des étapes de congélation/décongélation permet d'obtenir suffisamment de matériel biologique pour faire des expériences exigeant beaucoup de cellules (> 1-2.10⁷ cellules par point). En particulier et pour la première fois, il est possible d'amplifier les cellules d'un clone rendu silencieux pour un gène (*KIN17* dans cet exemple) par l'utilisation de vecteur selon la description et d'analyser *in vitro* ses activités de réparation de l'ADN.

Dans la figure 16, des essais préliminaires montrant la faisabilité de cette approche ont été réalisés. Il est à noter que cette approche n'est pas possible après une transfection transitoire de duplex siRNA ou de vecteurs intégratifs dans le sens où il faudrait transfecter énormément de cellules (très coûteux) et qu'il serait très difficile d'obtenir des clones stables.

L'utilisation de vecteurs selon la description permet donc pour la première fois d'analyser l'activité de complexes multi-protéiques actifs *in vitro* dans des clones (ou populations) isogéniques dérivant seulement par la perte (presque totale) de l'expression d'un gène essentiel. En effet, avec les vecteurs selon la description cela peut être entrepris rapidement, par exemple 15 jours après la transfection c'est-à-dire au moment où l'on observe déjà une très forte homogénéité des populations cellulaires en sélection. Cette cinétique dépend bien sûr du gène que l'on désire « éteindre » : par exemple cela peut être fait très rapidement après la transfection et la sélection hygromycine B dans le cas de XPA (exemple de *silencing* décrits plus haut). Cette approche peut bien sûr être entreprise après l'établissement des clones parce qu'ils sont stables (par exemple XPA, XPC ou KIN17).

### EXEMPLE 6 : vecteurs selon la description.

### 1. Construction des vecteurs de clonage

- Vecteur pBD751 : Le fragment « promoteur T7-LacZ' » du vecteur p205GTI (Stary et al., J Virol. 1989 Sep;63(9):3837-43.) a été amplifié par PCR avec les primers suivants : 5' GGT ACC AGA TCT GAT CCG AAA TTA 3 (SEQ ID NO:16) (contenant un site Bgl II) et 5' GTC GAC AAG CTT TTG ATC AGA TCG GTG CGG (SEQ ID NO : 17) (contenant un site Hind III), puis digéré par Bgl II / Hind III et introduit dans le vecteur pBD631 lui-même digéré par Bgl II / Hind III.
- Vecteur pBD889 : le fragment « PuroR + promoteur SV40 » proviennent du vecteur de De la Luna et al. (Gene 62, 121-126, 1988, Efficient transformation of mammalian cells with constructs containing a puromycin-resistance marker) dans lequel le site Hind III situé entre le promoteur SV40 et le gène de résistance à la puromycine (*PuroR*) a été délété par digestion Hind III et traitement par le fragment Klenow suivi d'une religation. Le vecteur obtenu a été appelé pBD881. Le vecteur pBD881 a été digéré par les enzymes de restriction BamH I / EcoR I pour prélever le fragment PuroR ; lequel a été introduit dans un vecteur EBV, appelé pBD152 et dérivé du vecteur pBD149 déjà cité ; ce vecteur comporte toute la séquence EBV (EBNA-1 + l'origine de réplication). Ce nouveau vecteur (pBD883 ou pEBV-Puro) a été digéré par les enzymes de restriction EcoR V / BamH I pour récupérer le fragment contenant la partie EBV, la partie bactérienne et la séquence promoteur SV40-PuroR ; ce fragment a été ligué avec le fragment EcoR V / BamH I du vecteur pBD650 pour créer le vecteur contrôle pBD886 ou pEBVsiControIR-Puro. Enfin, le vecteur pBD886 a été digéré par les enzymes de restriction Hind III / BamH I pour introduire le fragment de clonage H1 promoteur-T7-LacZ' du vecteur pBD751 ; ce nouveau vecteur a été appelé pBD899 ou pEBVsiR-LacZ'-Puro.

### 2. Construction du vecteur vide pBD884 (pEBVsiR-Puro)

Le vecteur pBD884 a été obtenu par digestion Spe I/BamH I du vecteur pBD883 (purification de la partie EBV-Puro) et ligation avec le fragment Spe I/BamH I du vecteur pBD631 (H1-sites de clonage Bgl II/Hind III). Le vecteur pBD884 est donc l'homologue du vecteur pBD631, le premier portant la résistance à l'hygromycine B et le second portant la résistance à la puromycine.

### 3. Résultats

La figure 17 montre l'intérêt de ces vecteurs de clonage. L'insertion des oligonucléotides (64-mer) dans le vecteur pBD751 (ou pBD899) est obtenue avec une efficacité proche de 100%, ce qui réduit considérablement le nombre de clones bactériens à analyser. Habituellement, 3 à 6 clones bactériens (colonies « blanches ») sont prélevés au hasard et leurs ADN plasmidiques sont isolés et analysés. Dans la plupart des cas, ces clones portent le plasmide recombinant (Figure 17C). Les efficacités de clonage sont extrêmement reproductibles.

### EXEMPLE 7 : Extinction des gènes DNA-PKcs et XRCC4

Dans la nature, il n'existe pas de mutant humain pour la protéine XRCC4. Pour la première fois, l'obtention de clones « mutants » dans lesquels le niveau d'expression de la protéine XRCC4 est extrêmement faible (environ 10% du niveau normal) est décrit ; en parallèle, un clone où la protéine DNA PKcs, partenaire de XRCC4 dans les voies de la recombinaison NHEJ et V(D)J, n'est plus détectable avec les techniques utilisées (marquages immunocytochimiques, western blot) est également décrit. Ces clones sont maintenus en culture depuis plus de 130 jours. Parallèlement à la réduction très importante du niveau de ces deux protéines dans les cellules HeLa en présence des vecteurs pBD694 et pBD743, la perte des activités spécifiques de ces protéines est démontrée : la Figure 19 démontre la perte totale d'activité DNA PKcs dans le clone DNA PKcs^{KD} et la Figure 20 démontre la perte de l'activité de réparation de l'ADN des clones XRCC4^{KD} et DNA PKcs^{KD} dans un test *in vitro* spécifique de la recombinaison NHEJ.

Les figures 18 à 20 montrent donc l'intérêt de ces vecteurs dans l'extinction à très long terme des protéines DNA-PKcs et XRCC4 associée à la perte de l'activité biologique correspondante.

### EXEMPLE 8 : Extinction des gènes p53, hRad50, hHR23A et hHR23B.

D'autres gènes d'intérêt ont été étudiés comme cibles des vecteurs pEBVsiRNA selon la description comme les gènes p53, hRad50, hHR23A et hHR23B. La figure 21 démontre que des vecteurs pEBVsiRNA spécifiques de ces gènes selon la description sont également très efficaces dans les cellules HeLa. Il ne semble donc pas y avoir de limitation sur la nature des gènes ciblés.

### EXEMPLE 9 : Extinction de l'expression des gènes à court, moyen et long terme.

La figure 22 résume différentes expériences menées de concert et démontre l'efficacité des vecteurs pEBVsiRNA selon la description à éteindre l'expression de gènes spécifiques (ici XPA, KIN17, XPC, XRCC4) à différents temps après la transfection (et la mise en sélection hygromycine B) des cellules HeLa. Ces vecteurs sont donc extrêmement efficaces à court, moyen et très long terme.

### RÉFÉRENCES

1. McManus M.T. et al., Nat Rev Genet, 2002, 3, 737-47.
2. Schwarz D.S. et al., Cell, 2003, 115, 199-208.
3. Elbashir S.M. et al., Nature, 2001, 411, 494-8.
4. Hasuwa H. et al., FEBS Lett, 2002, 532, 227-30.
5. Brummelkamp T.R. et al., Science, 2002, 296, 550-3.
6. Anonymous, Nat. Cell. Biol., 2003, 5, 489-490.
7. Butz K. et al., Oncogene, 2003, 22, 5938-45.
8. Laposa R.R. et al., Cancer Res, 2003, 63, 3909-12.
9. Paddison P.J. et al., Genes Dev, 2002, 16, 948-58.
10. Biard D.S. et al., Exp Cell Res, 1992, 200, 263-71.
11. Calos M.P., Trends Genet, 1996, 12, 463-6.
12. Okuda Y. et al., DNA Repair (Amst), 2004, 3, 1285-95.
13. Wakasugi M. et al., J. Biol. Chem., 1999, 274, 18759-68.
14. Masson C. et al., Proc. Natl. Acad. Sci. USA, 2003, 616-621.
15. Angulo J.F. et al., DNA Conformation and Transcription (E. Takashi, Ed.), Landes Biosciences, Ohyama, Konan University, 2004.
16. Khvorova A. et al., Cell, 2003, 115, 209-16.
17. Tuschl T., Chembiochem, 2001, 2, 239-45.
18. Chalk A.M. et al., Biochem Biophys Res Commun, 2004, 319, 264-74.
19. Scacheri P.C. et al., Proc Natl Acad Sci USA, 2004, 101, 1892-7.
20. Collins C.M. et al., Adv Cancer Res, 2002, 84, 155-74.
21. Dhar S.K. et al., Cell, 2001, 106, 287-296.
22. Chaudhuri B. et al., Proc Natl Acad Sci USA, 2001, 98, 10085-9.
23. Schepers A. et al., Embo J, 2001, 20, 4588-602.
24. Kapoor P. et al., J Virol, 2003, 77, 6946-56.
25. Friedberg E.C. et al., DNA Repair and Mutagenis, ASM Press, 1995.
26. Muotri A.R. et al., Carcinogenisis, 2002, 23, 1039-46.
27. Soutschek J. et al., Nature, 2004, 432, 173-178.
28. Bantounas I. et al., J. Mol. Endocrinol., 2004, 33, 545-557.
29. Dykxhoorn D. et al., Nature Reviews, 2003, 4, 457-467.
30. Barquinero J. et al., Gene Therapy, 2004, 11, 53-59.
31. Gilmore I.R. et al., J. Drug Targenting, 2004, 12, 6, 315-340.
32. Biard D.S. et al., BBA, 1992, 1130, 68-74.
33. Biofutur , 2002, 228, 52-61.
34. Voorhoeve et al., TIBS, 2003, 21, 2-4.
35. Sui G. et al., Proc Natl Acad Sci USA, 99, 5515-5520.
36. Dubois-Dauphin M. et al., J. Comp. Neurol., 2004, 474, 108-122.
37. Hu B. et al., Virol. Methods, 2004, 117, 129-136.
38. Liu BH. et al., Gene Ther., 2004, 11, 52-60.
39. Sato M. et al., Mol. Ther., 2003, 8, 726-737.
40. Yates et al., PNAS, 1984, 81, 3806-3810.
41. Sugden et al., Mol. Cell. Biol., 1985, 5, 410-413.
42. Rawlins et al., Cell, 1985, 42, 859-868.
43. Reisman et al., Mol. Cell. Biol., 1986, 6, 3838-3846.
44. Lupton et al., Mol. Cell. Biol., 1985, 5, 2533-2542.
45. Reisman et al., Mol. Cell. Biol., 1985, 5, 1822-1832.
46. Wysokenski et al., J. Virol., 1989, 63, 2657-2666.
47. Chittenden et al., J. Virol., 1989, 63, 3016-3025.
48. Krysan et al., Mol. Cell. Biol., 1991, 11, 1464-1472.
49. Krysan et al., Mol. Cell. Biol., 1989, 9, 1026-1033.
50. Gahn et al., Cell, 1989, 58, 527-535.
51. Sexton et al., J. Virol., 1989, 63, 5505-5508.
52. Chalk AM et al. , NAR, 2005, Database Issue, D131-4.
53. Yang ZG et al., Biochemistry, 2002, 41, 13012-13020.
54. Volker M. et al., Mol. Cell., 2001, 8, 213-224.
55. Sarasin et al., Br. J. Dermatol., 1992, 127, 485-491.
56. Daza et al., Biol. Chem., 1996, 377, 775-786.
57. Yates et al., Nature, 1985, 313, 812-815.
58. Biard D. et al., J. Biol. Chem., 2002, 277, 19156-65.

## Revendications

1. Vecteur d'expression d'un siRNA, capable d'inhiber ou d'éteindre l'expression d'un gène cible dans une cellule de mammifère, lequel vecteur est **caractérisé en ce qu'**il comprend :
(1) une cassette bactérienne comprenant une origine de réplication bactérienne et un marqueur de sélection bactérienne M1,
(2) une cassette de sélection dans les cellules eucaryotes comprenant un marqueur de sélection M2 de cellules eucaryotes et notamment de cellules de mammifère, sous le contrôle d'un promoteur approprié,
(3) une cassette EBV comprenant : (i) au moins un fragment de l'antigène nucléaire 1 du virus d'Epstein-Bar (EBNA-1) dont la séquence dérive du vecteur p205 dont 700 des 717 pb de la répétition Gly-Gly-Ala du virus B95-8 ont été délétés, (ii) au moins un fragment de la famille de répétition (FR) comprenant entre 6 et 20 copies du motif de 30 pb, chaque copie contenant un site d'association à la protéine virale EBNA-1, et des éléments de terminaison primaire de la réplication et (iii) la région de double symétrie (DYAD) et
(4) une cassette de transcription d'un siRNA comprenant au moins une région codant pour un siRNA correspondant au gène cible à inhiber ou à éteindre, sous le contrôle d'éléments régulateurs de transcription dans des cellules de mammifère, lesquels éléments régulateurs incluent au moins un promoteur capable de transcrire une séquence ADN codant pour un siRNA dans des cellules de mammifère et un terminateur de transcription ; ladite cassette de transcription est telle que la séquence codant pour ledit siRNA est immédiatement en aval du site d'initiation de la transcription ;
ledit vecteur étant **caractérisé en ce que** :
- ladite cassette de transcription d'un siRNA (4) comprend un promoteur H1,
- ladite région codant pour le siRNA est un shRNA, et
- ladite cassette de transcription d'un siRNA (4) et la cassette EBV (3) sont en orientation inverse.

2. Vecteur selon la revendication 1, **caractérisé en ce que** la cassette bactérienne (1) comprend un marqueur de sélection bactérienne M1 sélectionné dans le groupe constitué par les marqueurs de résistance à un antibiotique et les marqueurs d'auxotrophie.

3. Vecteur selon la revendication 1 ou la revendication 2, **caractérisé en ce que** la cassette de sélection dans les cellules eucaryotes (2) comprend un marqueur de sélection eucaryote M2 sélectionné dans le groupe constitué par :
- l'hygromycine et
- la puromycine.

4. Vecteur selon la revendication 3, **caractérisé en ce que** ledit marqueur de sélection M2 est sous le contrôle d'un promoteur convenable, sélectionné dans le groupe constitué par le promoteur de la thymidine kinase du virus HSV et le promoteur du virus SV40.

5. Cellules eucaryotes, de préférence cellules de mammifère, **caractérisées en ce qu'**elles contiennent un vecteur selon l'une quelconque des revendications 1 à 4.

6. Animal transgénique non humain, **caractérisé en ce qu'**il comprend des cellules modifiées selon la revendication 5.

7. Utilisation *in vitro* d'un vecteur selon l'une quelconque des revendications 1 à 4, pour inhiber ou éteindre l'expression d'un gène.

8. Composition pharmaceutique, **caractérisée en ce qu'**elle comprend un vecteur selon l'une quelconque des revendications 1 à 4 et au moins un véhicule pharmaceutiquement acceptable.

9. Procédé d'évaluation de l'activité d'un siRNA apte à inhiber ou éteindre l'expression d'un gène cible, lequel procédé est **caractérisé en ce qu'**il comprend les étapes suivantes:
(a₀) la transfection d'au moins une cellule de mammifère avec un vecteur selon l'une quelconque des revendications 1 à 4,
(b₀) la mise en culture desdites cellules dans un milieu apte à sélectionner les cellules résistantes au marqueur de sélection eucaryote M2 et
(c₀) l'analyse du phénotype desdites cellules résistantes par comparaison avec le phénotype de cellules non transfectées.

10. Procédé d'évaluation de l'activité d'un ensemble de siRNA conçus pour inhiber ou éteindre l'expression d'un gène cible, lequel procédé comprend:
(a₁) la préparation d'une banque de vecteurs selon l'une quelconque des revendications 1 à 4, dans lesquels différents siRNA, ayant le même gène cible, sont insérés,
(b₁) la transfection de cellules avec lesdits vecteurs de ladite banque; chaque cellule ou chaque série de cellules étant transfectée avec un vecteur différent,
(c₁) la mise en culture desdites cellules dans un milieu apte à sélectionner les cellules résistantes au marqueur de sélection eucaryote M2, et
(d₁) l'analyse des différents phénotypes desdites cellules résistantes.

11. Procédé selon la revendication 9 ou la revendication 10, **caractérisé en ce que** l'étape (c₀) ou l'étape (d₁) d'analyse du phénotype comprend la détection et/ou la quantification de l'expression du gène cible.

12. Procédé selon l'une quelconque des revendications 9 à 11, **caractérisé en ce qu'**il comprend en outre, après l'étape (c₀) ou après l'étape (d₁), une étape (c'₀) ou une étape (d'₁) de mise en culture desdites cellules dans un milieu dépourvu de l'élément permettant la sélection des cellules résistantes au marqueur de sélection M2 et l'évaluation de la restauration du phénotype de départ.

13. Procédé de criblage de molécules aptes à traiter une maladie comprenant l'inhibition ou l'extinction d'un gène exprimant une protéine, lequel procédé comprend :
(a₂) la préparation d'un modèle d'étude de ladite maladie par la transfection d'au moins une cellule de mammifère avec un vecteur selon l'une quelconque des revendications 1 à 4, comprenant un siRNA capable de mimer ladite maladie,
(b₂) la mise en en culture desdites cellules dans un milieu apte à sélectionner les cellules résistantes au marqueur de sélection eucaryote M2,
(c₂) l'analyse du phénotype desdites cellules résistantes par comparaison avec le phénotype desdites cellules avant transfection,
(d₂) l'ajout dans le milieu de culture de la substance ou de la banque de substances à cribler,
(e₂) l'évaluation de la modification du phénotype desdites cellules et
(f₂) la sélection des molécules restaurant un phénotype normal et donc aptes à traiter ladite maladie.

14. Utilisation d'une cellule selon la revendication 5, pour évaluer l'activité globale d'un siRNA.

15. Kit, **caractérisé en ce qu'**il comprend au moins un vecteur selon l'une quelconque des revendications 1 à 4 ou des cellules selon la revendication 5 et des moyens de détection et/ou de quantification de l'expression d'un gène cible.

16. Produit intermédiaire pour la préparation d'un vecteur selon l'une quelconque des revendications 1 à 4, **caractérisé en ce qu'**il comprend :
(1) une cassette bactérienne comprenant une origine de réplication bactérienne et un marqueur de sélection bactérienne M1, telle que définie à la revendication 1 ou à la revendication 2,
(2) une cassette de sélection dans les cellules eucaryotes comprenant un marqueur de sélection M2 de cellules eucaryotes et notamment de cellules de mammifère, sous le contrôle d'un promoteur approprié, telle que définie à l'une quelconque des revendications 1 à 4,
(3) une cassette EBV comprenant (i) au moins un fragment de l'antigène nucléaire 1 du virus d'Epstein-Bar (EBNA-1) dont la séquence dérive du vecteur p205 dont 700 des 717 pb de la répétition Gly-Gly-Ala du virus B95-8 ont été délétés, (ii) au moins un fragment de la famille de répétition (FR) comprenant entre 6 et 20 copies du motif de 30 pb, chaque copie contenant un site d'association à la protéine virale EBNA-1, et des éléments de terminaison primaire de la réplication, et (iii) la région de double symétrie (DYAD), et
(4) le promoteur H1, suivi de deux sites de clonage, orienté en sens inverse par rapport à la direction de la cassette EBV.

17. Produit intermédiaire selon la revendication 16, **caractérisé en ce que** les deux sites de clonage sélectionnés sont Bgl II et Hind III.

18. Produit intermédiaire selon la revendication 16 ou la revendication 17, **caractérisé en ce que** le marqueur de sélection M2 est l'hygromycine, ledit produit intermédiaire étant dénommé pBD631 ou pEBV-siR.

19. Produit intermédiaire selon la revendication 16 ou la revendication 18, **caractérisé en ce qu'**il comprend un système de sélection pour le clonage comprenant un promoteur procaryote (T7) suivi du fragment alpha du gène LacZ (LacZ').

20. Produit intermédiaire selon la revendication 19, **caractérisé en ce que** le marqueur de sélection M2 est l'hygromycine, ledit produit intermédiaire étant dénommé pBD751 ou pEBVsiR-LacZ' (ou pEBVsiR-VacZ'-Hygro).

21. Produit intermédiaire selon la revendication 17, **caractérisé en ce que** le marqueur de sélection M2 est la puromycine, ledit produit intermédiaire étant dénommé pBD899 ou pEBVsiR-LacZ'-Puro.

22. Procédé de préparation d'un vecteur selon l'une quelconque des revendications 1 à 4, **caractérisé en ce qu'**il comprend :
- la préparation d'un produit intermédiaire, selon l'une quelconque des revendications 16 à 21 et
- l'insertion d'une séquence d'ADN codant un shRNA convenable, au niveau de deux sites de clonage sélectionnés.

## Patentansprüche

1. siRNA-Expressionsvektor, der in der Lage ist, die Expression eines Zielgens in einer Säugetierzelle zu hemmen oder auszuschalten, wobei der Vektor **dadurch gekennzeichnet ist, dass** er Folgendes umfasst:
(1) eine bakterielle Kassette, die einen bakteriellen Replikationsursprung und einen Marker M1 zur bakteriellen Selektion umfasst,
(2) eine Selektionskassette in den Eukaryotenzellen, die einen Selektionsmarker M2 von Eukaryotenzellen und insbesondere von Säugetierzellen, unter der Kontrolle eines geeigneten Promotors, umfasst
(3) eine EBV-Kassette, die Folgendes umfasst: (i) mindestens ein Fragment des Kernantigens 1 des Epstein-Barr-Virus (EBNA-1), dessen Sequenz vom Vektor p205 abgeleitet ist, wovon 700 der 717 Basenpaare von der Wiederholung Gly-Gly-Ala des Virus B95-8 deletiert wurden, (ii) mindestens ein Fragment der Wiederholungsfamilie (FR), die zwischen 6 und 20 Kopien des Motivs von 30 Basenpaaren umfasst, wobei jede Kopie eine Bindungsstelle für das virale Protein EBNA-1 und primäre Terminationselemente der Replikation enthält, und (iii) die Doppelsymmetrieregion (DYAD) und
(4) eine Transkriptionskassette einer siRNA, die mindestens eine codierende Region für eine siRNA umfasst, die dem zu hemmenden oder auszuschaltenden Zielgen entspricht, unter der Kontrolle von Transkriptionsregulatorelementen in Säugetierzellen, wobei die Regulatorelemente mindestens einen Promotor, der in der Lage ist, eine codierende DNA-Sequenz für eine siRNA in Säugetierzellen zu transkribieren, und einen Transkriptionsterminator umfassen; wobei die Transkriptionskassette derart ist, dass die codierende Sequenz für die siRNA der Initiierungsstelle der Transkription unmittelbar vorgeschaltet ist;
wobei der Vektor **dadurch gekennzeichnet ist, dass**:
- die Transkriptionskassette einer siRNA (4) einen H1-Promotor umfasst,
- die codierende Region für die siRNA eine shRNA ist, und
- die Transkriptionskassette einer siRNA (4) und die EBV-Kassette (3) sich in umgekehrter Ausrichtung befinden.

2. Vektor nach Anspruch 1, **dadurch gekennzeichnet, dass** die bakterielle Kassette (1) einen Marker M1 zur bakteriellen Selektion umfasst, der aus der Gruppe ausgewählt ist, die aus einem Marker für eine Antibiotikaresistenz und Auxotrophiemarkern besteht.

3. Vektor nach Anspruch 1 oder Anspruch 2, **dadurch gekennzeichnet, dass** die Selektionskassette in den Eukaryotenzellen (2) einen Eukaryoten-Selektionsmarker M2 umfasst, der aus der Gruppe ausgewählt ist, die aus Folgendem besteht:
- Hygromicin und
- Puromycin.

4. Vektor nach Anspruch 3, **dadurch gekennzeichnet, dass** der Selektionsmarker M2 unter der Kontrolle eines angemessenen Promotors ist, der aus der Gruppe ausgewählt ist, die aus dem Promotor der Thymidinkinase des HSV-Virus und dem Promotor des SV40-Virus besteht.

5. Eukaryotenzellen, vorzugsweise Säugetierzellen, **dadurch gekennzeichnet, dass** sie einen Vektor nach einem der Ansprüche 1 bis 4 enthalten.

6. Nicht menschliches transgenes Tier, **dadurch gekennzeichnet, dass** es modifizierte Zellen nach Anspruch 5 umfasst.

7. In-vitro-Verwendung eines Vektors nach einem der Ansprüche 1 bis 4 zum Hemmen oder Ausschalten der Expression eines Gens.

8. Pharmazeutische Zusammensetzung, **dadurch gekennzeichnet**, das sie einen Vektor nach einem der Ansprüche 1 bis 4 und mindestens einen pharmazeutisch annehmbaren Träger umfasst.

9. Verfahren zur Bewertung der Aktivität einer siRNA, die in der Lage ist, die Expression eines Zielgens zu hemmen oder auszuschalten, wobei das Verfahren **dadurch gekennzeichnet ist, dass** es die folgenden Schritte umfasst:
(aₒ) die Transfektion von mindestens einer Säugetierzelle mit einem Vektor nach einem der Ansprüche 1 bis 4,
(b₀) die Kultivierung der Zellen in einem Medium, das sich dazu eignet, die gegenüber dem Eukaryoten-Selektionsmarker M2 resistenten Zellen zu selektieren und
(c₀) die Analyse des Phänotyps der resistenten Zellen durch Vergleich mit dem Phänotyp von nicht transfektierten Zellen.

10. Verfahren zur Bewertung der Aktivität einer Menge von siRNA, die zugeschnitten sind, die Expression eines Zielgens zu hemmen oder auszuschalten, wobei das Verfahren Folgendes umfasst:
(a₁) die Vorbereitung einer Bank von Vektoren nach einem der Ansprüche 1 bis 4, in die verschiedene siRNA, die das gleiche Zielgen aufweisen, eingefügt werden,
(b₁) die Transfektion von Zellen mit den Vektoren der Bank; wobei jede Zelle oder jede Reihe von Zellen mit einem unterschiedlichen Vektor transfektiert wird,
(c₁) die Kultivierung der Zellen in einem Medium, das geeignet ist, die gegenüber dem Eukaryoten-Selektionsmarker M2 resistenten Zellen zu selektieren, und
(d₁) die Analyse der verschiedenen Phänotypen der resistenten Zellen.

11. Verfahren nach Anspruch 9 oder Anspruch 10, **dadurch gekennzeichnet, dass** der Schritt (c₀) oder der Schritt (d₁) zur Analyse des Phänotyps die Detektion und/oder die Quantifizierung der Expression des Zielgens umfasst.

12. Verfahren nach einem der Ansprüche 9 bis 11, **dadurch gekennzeichnet, dass** es ferner nach dem Schritt (c₀) oder nach dem Schritt (d₁) einen Schritt (c'₀) oder einen Schritt (d'₁) zum Kultivieren der Zellen in einem Medium ohne das Element umfasst, das die Selektion der gegenüber dem Selektionsmarker M2 resistenten Zellen und die Bewertung der Wiederherstellung des Ausgangsphänotyps ermöglicht.

13. Verfahren zum Screening von Molekülen, die geeignet sind, eine Krankheit zu behandeln, das die Hemmung oder Ausschaltung eines Gens umfasst, das ein Protein exprimiert, wobei das Verfahren Folgendes umfasst:
(a₂) die Vorbereitung eines Studienmodells der Krankheit durch die Transfektion von mindestens einer Säugetierzelle mit einem Vektor nach einem der Ansprüche 1 bis 4, die eine siRNA umfasst, die in der Lage ist, die Krankheit nachzuahmen,
(b₂) die Kultivierung der Zellen in einem Medium, das geeignet ist, die gegen den Eukaryotenselektionsmarker M2 resistenten Zellen zu selektieren,
(c₂) die Analyse des Phänotyps der resistenten Zellen durch Vergleich mit dem Phänotyp der Zellen vor der Transfektion,
(d₂) das Beigeben der zu screenenden Substanz oder Bank von Substanzen zu dem Medium,
(e₂) die Bewertung der Modifikation des Phänotyps der Zellen und
(f₂) die Selektion der Moleküle, die einen normalen Phänotyp wiederherstellen und somit geeignet sind, die Krankheit zu behandeln.

14. Verwendung einer Zelle nach Anspruch 5 zum Bewerten der globalen Aktivität einer siRNA.

15. Kit, **dadurch gekennzeichnet, dass** es mindestens einen Vektor nach einem der Ansprüche 1 bis 4 oder Zellen nach Anspruch 5 und Mittel zur Detektion und/oder Quantifizierung der Expression eines Zielgens umfasst.

16. Zwischenprodukt zur Vorbereitung eines Vektors nach einem der Ansprüche 1 bis 4, **dadurch gekennzeichnet, dass** es Folgendes umfasst:
(1) eine bakterielle Kassette, die einen bakteriellen Replikationsursprung und einen Marker M1 zur bakteriellen Selektion umfasst, nach Anspruch 1 oder Anspruch 2,
(2) eine Selektionskassette in den Eukaryotenzellen, die einen Selektionsmarker M2 von Eukaryotenzellen und insbesondere von Säugetierzellen unter der Kontrolle eines geeigneten Promotors umfasst, nach einem der Ansprüche 1 bis 4,
(3) eine EBV-Kassette, die Folgendes umfasst: (i) mindestens ein Fragment des Kernantigens 1 des Epstein-Barr-Virus (EBNA-1), dessen Sequenz vom Vektor p205 abgeleitet ist, wovon 700 der 717 Basenpaare von der Wiederholung Gly-Gly-Ala des Virus B95-8 deletiert wurden, (ii) mindestens ein Fragment der Wiederholungsfamilie (FR), die zwischen 6 und 20 Kopien des Motivs von 30 Basenpaaren umfasst, wobei jede Kopie eine Bindungsstelle für das virale Protein EBNA-1 und primäre Terminationselemente der Replikation enthält, und (iii) die Doppelsymmetrieregion (DYAD) und
(4) den Promotor H1, gefolgt von zwei Klonierungsstellen, der in Bezug auf die Richtung der EBV-Kassette in entgegengesetzter Richtung ausgerichtet ist.

17. Zwischenprodukt nach Anspruch 16, **dadurch gekennzeichnet, dass** die zwei selektierten Klonierungsstellen Bgl II und Hind III sind.

18. Zwischenprodukt nach Anspruch 16 oder Anspruch 17, **dadurch gekennzeichnet, dass** der Selektionsmarker M2 Hygromycin ist, wobei das Zwischenprodukt als pBD631 oder pEVB-siR bezeichnet wird.

19. Zwischenprodukt nach Anspruch 16 oder Anspruch 18, **dadurch gekennzeichnet, dass** es ein Selektionssystem für die Klonierung umfasst, das einen Prokaryot-Promotor (T7), gefolgt von einem Alpha-Fragment des Gens LacZ (LacZ'), umfasst.

20. Zwischenprodukt nach Anspruch 19, **dadurch gekennzeichnet, dass** der Selektionsmarker M2 Hygromycin ist, wobei das Zwischenprodukt als pBD751 oder pEBVsiR-LacZ' (oder pEBVsiR-VacZ'-Hygro) bezeichnet wird.

21. Zwischenprodukt nach Anspruch 17, **dadurch gekennzeichnet, dass** der Selektionsmarker M2 Puromycin ist, wobei das Zwischenprodukt als pBD899 oder pEBVsiR-LacZ'-Puro bezeichnet wird.

22. Verfahren zur Vorbereitung eines Vektors nach einem der Ansprüche 1 bis 4, **dadurch gekennzeichnet, dass** es Folgendes umfasst:
- die Vorbereitung eines Zwischenprodukts nach einem der Ansprüche 16 bis 21 und
- die Einfügung einer codierenden DNA-Sequenz, die eine angemessene shRNA codiert, im Bereich von zwei selektionierten Klonierungsstellen.

## Claims

1. An siRNA expression vector capable of inhibiting or extinguishing the expression of a target gene in a mammalian cell, which vector is **characterized in that** it comprises:
(1) a bacterial cassette comprising a bacterial origin of replication and a bacterial selectable marker M1,
(2) a cassette for selection in eukaryotic cells comprising a selectable marker M2 for eukaryotic cells, and in particular for mammalian cells, in particular mammalian cells, under the control of an appropriate promoter,
(3) an EBV cassette comprising: (i) at least one fragment of the Epstein-Barr virus nuclear antigen 1 (EBNA-1) that derives from the vector p205 and in which 700 of the 717 bp of the Gly-Gly-Ala repeat of the B95-8 virus have been deleted, (ii) at least one fragment of the family of repeats (FR) comprising between 6 and 20 repeats of the 30 bp motif, each copy containing a binding site for the EBNA-1 viral protein, and elements for primary termination of replication and (iii) the double symmetry (DYAD) region, and
(4) an siRNA transcription cassette comprising at least one region encoding an siRNA corresponding to the target gene to be inhibited or to be extinguished, under the control of regulatory elements for transcription in mammalian cells, which regulatory elements include at least one promoter capable of transcribing an siRNA in mammalian cells and a transcription terminator; said transcription cassette is immediately downstream of the transcription initiation site;
Said vector being **characterized in that**:
- Said siRNA transcription cassette (4) contains a H1 promoter,
- said region encoding an siRNA is a shRNA, and
- said siRNA transcription cassette (4) and EBV cassette (3) are in reverse orientation.

2. The vector as claimed in claim 1, **characterized in that** the bacterial cassette (1) comprises a bacterial selectable marker M1 selected from the group consisting of markers for resistance to an antibiotic and auxo-trophic markers.

3. The vector as claimed in claim 1 or claim 2, **characterized in that** the cassette for selection in eukaryotic cells (2) comprises a eukaryotic selectable marker M2 selected from the group consisting of:
- hygromycin, and
- puromycin.

4. The vector as claimed in claim 3, **characterized in that** said selectable marker M2 is under the control of a suitable promoter, selected from the group consisting of the HSV thymidine kinase promoter and the SV40 promoter.

5. A eukaryotic cell, preferably a mammalian cell, **characterized in that** it contains a vector as claimed in any one of claims 1 to 4.

6. A nonhuman transgenic animal, **characterized in that** it comprises modified cells as claimed in claim 5.

7. The *in vitro* use of a vector as claimed in any one of claims 1 to 4, for inhibiting or extinguishing the expression of a gene.

8. A pharmaceutical composition, **characterized in that** it comprises a vector as claimed in any one of claims 1 to 4, and at least one pharmaceutically acceptable carrier.

9. A method for evaluating the activity of an siRNA capable of inhibiting or extinguishing the expression of a target gene, which method is **characterized in that** it comprises the following steps:
(a₀) transfecting at least one mammalian cell with a vector as claimed in any one of claims 1 to 4,
(b₀) culturing said cells in a medium capable of selecting the cells resistant to the eukaryotic selectable marker M2, and
(c₀) analyzing the phenotype of said resistant cells by comparison with the phenotype of nontransfected cells.

10. A method for evaluating the activity of a collection of siRNAs designed so as to inhibit or extinguish the expression of a target gene, which method comprises:
(a₁) preparing a library of vectors as claimed in any one of claims 1 to 4 into which various siRNAs, having the same target gene, are inserted,
(b₁) transfecting cells with said vectors of said library; each cell or each series of cells being transfected with a different vector,
(c₁) culturing said cells in a medium capable of selecting the cells resistant to the eukaryotic selectable marker M2, and
(d₁) analyzing the various phenotypes of said resistant cells.

11. The method as claimed in claim 9 or claim 10, **characterized in that** step (c₀) or step (d₁) for analyzing the phenotype comprises the detection and/or the quantification of the expression of the target gene.

12. The method as claimed in any one of claims 9 to 11, **characterized in that** it also comprises, after step (c₀) or after step (d₁, a step (c'₀) or a step (d'₁) for culturing said cells in a medium devoid of the element for selecting the cells resistant to the selectable marker M2 and evaluating the restoration of the starting phenotype.

13. A method for screening for molecules capable of treating a disease, comprising the inhibition or the extinction of a gene expressing a protein, which method comprises:
(a₂) preparing a model for studying said disease by transfecting at least one mammalian cell with a vector as claimed in any one of claims 1 to 4, comprising an siRNA capable of mimicking said disease,
(b₂) culturing said cells in a medium capable of selecting the cells resistant to the eukaryotic selectable marker M2,
(c₂) analyzing the phenotype of said resistant cells by comparison with the phenotype of said cells before transfection,
(d₂) adding to the culture medium the substance or the library of substances to be screened,
(e₂) evaluating the modification of the phenotype of said cells, and
(f₂) selecting the molecules which will restore a normal phenotype and are therefore capable of treating said disease.

14. The use of a cell as claimed in claim 5, for evaluating the overall activity of an siRNA.

15. A kit, **characterized in that** it comprises at least one vector as claimed in any one of claims 1 to 4 or cells as claimed in claim 5, and means for detecting and/or quantifying the expression of a target gene.

16. An intermediate product for the preparation of a vector as claimed in any one of claims 1 to 4, **characterized in that** it comprises:
(1) a bacterial cassette comprising a bacterial origin of replication and a bacterial selectable marker M1, as defined in claim 1 or claim 2,
(2) a cassette for selection in eukaryotic cells, comprising a selectable marker M2 for eukaryotic cells, in particular for mammalian cells, under the control of an appropriate promoter, as defined in any one of claims 1 to 4,
(3) an EBV cassette comprising (i) at least one fragment of the Epstein-Barr virus nuclear antigen 1 (EBNA-1) that derives from the vector p205 and in which 700 of the 717 bp of the Gly-Gly-Ala repeat of the B95-8 virus have been deleted, (ii) at least one fragment of the family of repeats (FR) comprising between 6 and 20 repeats of the 30 bp motif, each copy containing a binding site for the EBNA-1 viral protein, and elements for primary termination of replication and (iii) at least one fragment of the double symmetry (DYAD) region, and
(4) the H1 promoter followed by two cloning sites selected, in the reverse orientation compared to the EBV cassette.

17. The intermediate product as claimed in claim 16, **characterized in that** the two cloning sites selected are Bgl II and Hind III.

18. The intermediate product as claimed in claim 16 or claim 17, **characterized in that** the selectable marker M2 is hygromycin, said intermediate product being called pBD665 or pEBV-siD.

19. The intermediate product as claimed in claim 16 or claim 18, **characterized in that** it comprises a selection system for the cloning, comprising a prokaryotic promoter (T7) followed by the alpha fragment of the LacZ gene (LacZ').

20. The intermediate product as claimed in claim 19, **characterized in that** the selectable marker M2 is hygromycin, said intermediate product being called pBD751 or pEBVsiR-LacZ' (or pEBVsiR-LacZ'-Hygro).

21. The intermediate product as claimed in claim 17, **characterized in that** the selectable marker M2 is puromycin, said intermediate product being called pBD899 or pEBVsiR-LacZ'-Puro.

22. A method for preparing a vector as claimed in any one of claims 1 to 4, **characterized in that** it comprises:
- preparing an intermediate product as claimed in any one of claims 16 to 21, and
- inserting a DNA sequence encoding a suitable shRNA into two selected cloning sites.
